# EUROPEAN PATENT APPLICATION

(11) **EP 4 779 635 A1**
(43) Date of publication of application: **22.07.2026**
(21) Application number: 26152190.0
(22) Date of filing: 15.01.2026
(51) Int. Cl.: G10L 25/66, A61B 5/00

(54) **METHOD OF AN AUTOMATED PROFILING OF SPEECH RESPONSES IN MAPPING AND REHABILITATION OF SPEECH AND SYSTEM THEREOF**

(30) Priority: 15.01.2025 GR 20250100027
(71) Applicant: Aristotle University Of Thessaloniki-Eidikos Logariasmos Kondilion Erevnas E.L.K.E., 54636 Thessaloniki (GR)
(72) Inventor: PASTIADIS, Konstantinos, 57001 Neo Rysio Thessaloniki (GR); KIMISKIDIS, Vasilios, 54454 Thessaloniki (GR); KUGIUMTZIS, Dimitrios, 54351 Thessaloniki (GR); LIOUMIS, Pantelis, 00570 Helsinki (FI)
(74) Representative: Petsis, Christos

(57) **Abstract**

The invention relates to a method for an automated profiling of speech responses in mapping and rehabilitation of speech based on the following steps consisting of
- performing automatically and fast with minimal human operator effort & intervention;
- extracting, quantifying, evaluating and assessing the subject's speech and/or its characteristics such as accuracy, quantification of diversions or adherence or conformance to the demanded task, namely errors, semantics, lexical, pragmatics, phonetics, timings, sequential characteristics, of the response) in relation to the offered visual, auditory, olfactory, tactile, vibratory, motional template and demanded task;
- performing classification, clustering and modeling of the content and signal characteristics of the sequences of events in the subject responses, producing appropriate utterance's and speaker's profiles;
- augmenting the analysis of the latter step by an appropriate feedback from previous utterance's/speaker's profiles, which are formed by accumulation of previous analyses or beforehand knowledge;
- maximizing and optimizing the acquired subject or population profile information through an adaptive coordination of the process; by means of
a set of proposed operations consisting of
- the Collection of subject responses, along with a variety of additional data;

- the Removal of any unwanted signals that may be mixed with the subject speech;
- the Performance of phonetical, phonological, lexical, semantic, and pragmatic NLP along/or after the speech's content recognition;
- the Performance of intelligent and adaptive coordination of execution flow and selection of the task/paradigm's presentation and interventional parameters;
- the Building of a system that is structured to be trainable and supervisable, esp. that recurrently accumulates, analyses and makes decisions upon its inputs and outputs, and thus constitutes an intelligent system.

## Description

### Field of the invention

The present invention relates to a method for an Automated, Adaptive and Intelligent Profiling of Speech Responses of a subject in Mapping, Diagnosis, Treatment and Rehabilitation thereof, as well as to a system therefor.

### Background of the invention

A synthetic overview of Speech and Language pathologies is set out hereafter. Various types of brain or peripheral pathologies may lead to disruption of speech production and perception. Morphosyntactic deficits, timing and neuromuscular coordination problems, alterations of voice qualities, have been identified as key symptoms of various such pathologies [1]-[6].

Aphasia such as anomia, paraphasia, dysarthria, and apraxia are all speech and language disorders that may result from different neurological conditions or injuries affecting the brain's language areas [7]. Each of these disorders involves specific difficulties in speech production or language processing.

These disorders can co-occur in some individuals or may be present alongside other communication and cognitive difficulties. Speech and language therapy is often recommended for individuals with these disorders to improve communication skills and enhance their quality of life. Treatment approaches may vary based on the specific challenges each person faces and the underlying cause of their speech and language difficulties.

In Motor Speech Production Assessment, Motor speech production testing evaluates the integrity and functionality of the speech motor system in individuals who may have both speech production and processing difficulties. This assessment is particularly important for diagnosing and understanding motor speech disorders, such as dysarthria and apraxia of speech [1]-[3], [8].

During motor speech production testing, a series of tasks may be employed to assess different aspects of speech production, including articulation, phonation, prosody and speech coordination. The specific tests and tasks used may vary depending on the suspected underlying condition and the individual's age and communication abilities.

The results of motor speech production testing help to determine the presence and nature of any motor speech disorders, leading to appropriate treatment planning. Treatment for motor speech disorders may include speech exercises, strategies to improve articulation and coordination, and techniques to enhance overall communication effectiveness. Regular follow-up assessments can also track progress and adjust treatment goals as needed.

In frameworks of complex tasks related to cognition and speech production, the previously cited Speech Motor investigations are usually employed within more complex controlled experimental procedures or activities in order to investigate specific cognitive, perceptual or motor functions of the brain. These tasks are designed to elicit and measure particular neural processes or behavioral responses to better understand how the brain works and how it is involved in various functions and behaviors.

Tasks can vary widely depending on the specific research question and the brain area or function being studied. Of specific interest are tasks related to voice/speech and language perception, processing and production, especially in clinical research or therapy frameworks. These tasks are designed to investigate various aspects of language processing, including comprehension, production and semantic processing. Participants might engage in tasks like reading sentences, naming objects, or generating words [6], [9]-[14].

Some of the most common tasks are Naming Tasks, Repetition Tasks & Narrative description Tasks. The Naming Task is a common experimental paradigm used in both speech perception and speech production research. It involves participants being presented with auditory or visual stimuli -usually words or pictures- and then being asked to produce the corresponding name for the stimulus they were presented.

Naming tasks are valuable tools for studying language processing, assessing language abilities, and identifying potential language disorders, examining the effects of brain injuries on speech production, and exploring the relationships between language and cognition as they provide valuable insights into a person's lexical access, semantic knowledge, word retrieval speed, and language processing abilities. In speech perception research, the Naming Task is often used to study how individuals process and recognize spoken words. Participants might listen to recordings of isolated words or sentences and then verbally produce the name of the word they heard. Factors of interest include response accuracy, response time, and potential errors made during the Naming Task to gain insights into the perceptual mechanisms involved in recognizing spoken words.

In speech production research, the Naming Task is used to examine how individuals retrieve and produce words from their mental lexicon. Participants are typically presented with pictures or written words and asked to name the objects or words they see. This task helps researchers understand how certain factors, such as word frequency, word length, and semantic context, influence the speed and accuracy of word retrieval and production.

Repetition Tasks are exercises commonly used in speech and language therapy to improve auditory processing, working memory, and speech production skills. These tasks involve listening to a series of spoken words or sentences and then repeating them back accurately. They can be beneficial for individuals with various speech and language difficulties, including children with language delays, individuals with speech sound disorders, and those with difficulties in processing and recalling auditory information.

Narrative Description Tasks involve presenting participants with written or spoken narratives and assessing their ability to understand and/or reproduce the content accurately. Researchers use these tasks to investigate various aspects of language processing and comprehension, as well as higher-level cognitive functions involved in constructing and understanding stories. The structure and complexity of narrative description tasks may vary depending on the research question and the targeted cognitive processes. Common types of narrative description tasks notably include Comprehension Tasks, Story Recall or Retelling Tasks, Story Completion Tasks, Emotional Narratives, Neural Correlates of Narrative Processing, Age and Developmental Studies.

There are Brain Stimulation approaches in cognitive and motor processes related to Speech, wherein Brain stimulation techniques have been used in research and clinical settings to investigate and modulate speech perception and production processes. These techniques involve applying electrical or magnetic impulses to specific brain regions to either enhance or inhibit their activity, providing valuable insights into the neural mechanisms underlying speech and language [15]-[31], [32], [33].

There are three commonly used brain stimulation techniques relevant to speech perception and production, such as Transcranial Magnetic Stimulation (TMS). These techniques have shown promise in uncovering the neural underpinnings of speech perception and production. Additionally, these brain stimulation techniques are being explored for their potential therapeutic applications in speech and language disorders, but more research is needed to fully understand their effectiveness and safety for clinical use. It is important to note that brain stimulation research in this field is continuously evolving, and any potential clinical applications must be conducted under the guidance of trained professionals and in compliance with ethical guidelines.

Additionally, regarding Rehabilitation for Speech-Language Disorders, the study of speech production and perception plays a crucial role in speech-language rehabilitation, as they are directly related to an individual's ability to communicate effectively. Speech-language rehabilitation aims to improve communication skills, overcome speech and language disorders, and enhance overall communication abilities [41]-[44]. There are Rehabilitation techniques aiming at Speech Production, & Speech Perception.

Regarding Automated processes in the field of Speech/Language Research and Therapy, in all paradigms regarding speech production/perception research and treatment setups, Automated Speech Recognition can greatly facilitate speed, accuracy and extraction of important features, which are not easily discernible to traditional human listening and judgement or quantification [45]-[51]. However, automated recognition and information mining from speech or speech-based responses are most often a challenging task, since several issues may arise:
Automated Speech Recognition (ASR) and speech analysis have made significant progress in recent years, but several challenges still exist, such as Variability in Speech, Background Noise, Speaker Segregation.

As to Variability in Speech, Speech exhibits substantial variability due to differences in accents, dialects, speaking rates, and individual speaking styles. ASR systems must be robust enough to handle this variability and accurately transcribe speech from diverse speakers, or any other type of variability stemming from speech production mechanisms or problems, such as the ones set out above.

As to Background Noise, Real-world environments often have background noise, such as crowd chatter, machinery sounds or music. Noise reduction and robust speech recognition techniques are required to effectively process speech in noisy conditions.

As to Speaker Segregation, In scenarios where multiple speakers are involved, such as meetings or group discussions, it becomes challenging to identify and separate each speaker's speech accurately. As to Out-of-Vocabulary (OOV) Words, the above-mentioned ASR systems are typically trained on a specific vocabulary, and they may struggle to recognize words that are not present in their training data. Handling OOV words is critical in ensuring accurate transcription.

As to Domain Adaptation, said ASR systems trained on one domain may not perform well in other domains due to differences in language use, terminology, and acoustics. Adapting ASR models to specific domains is essential for achieving high accuracy.

As to Disfluencies and Speech Errors, Human speech is often filled with disfluencies, hesitations, and corrections. Said ASR systems need to handle these interruptions and correctly transcribe the intended message.

As to Context and Co-reference, understanding the context and resolving co-reference in speech is challenging. It requires the ability to track and link pronouns and other expressions referring to their antecedents.

As to Limited Training Data, Training robust ASR models requires a large amount of labeled speech data, which might not always be available for specific languages or domains.

As to Online and Real-Time Processing, said ASR systems used in real-time applications, such as voice assistants or live transcriptions, need to process speech quickly and efficiently, leaving little room for processing delays.

As to Speaker and Language Adaptation, Personalized speech recognition for individual speakers or users is crucial for personalized applications, but it can be challenging to achieve high accuracy with limited personalized data.

Core components of Automated processes in the field of Speech/Language Research and Therapy are set out hereafter.

Acoustic scene analysis and segregation are two related tasks in the field of audio signal processing, particularly in the context of separating different sound sources from a mixture of sounds within an acoustic environment [12], [53]-[65].

Acoustic Scene Analysis (ASA) refers to the process of analyzing and understanding the auditory scene, i.e. the collection of sounds and acoustic events present in a given environment. The objective of said ASA is to extract meaningful information from the mixture of audio signals, such as identifying the sources, recognizing events, or understanding the context of the scene.

Acoustic Scene Segregation (ASS), also known as source separation or sound source separation, is the process of separating individual sound sources from a mixture of audio signals. The goal is to reconstruct the original signals of each sound source, given only the mixed audio signal.

Speech-to-text (STT) methods, also known as Automatic Speech Recognition (ASR) methods, convert spoken language into written text. Over the years, various approaches have been developed to perform this task [66]-[73]. Some common methods used in speech-to-text systems include:
Hidden Markov Models (HMM): HMMs have been a traditional approach for said ASR. They model the probability distribution of acoustic features (such as Mel-frequency cepstral coefficients, or MFCCs) over time. The underlying assumption is that the speech signal can be represented as a sequence of states, each associated with an acoustic observation;
Gaussian Mixture Models (GMM): GMMs are used in combination with HMMs in many older ASR systems. They are employed to model the probability distribution of the acoustic features in each HMM state.

Each of these methods has its strengths and weaknesses, and the choice of method depends on the specific requirements of the ASR application and the available resources. Deep learning approaches, especially those based on Transformers, are gaining popularity due to their ability to achieve state-of-the-art results across various speech recognition tasks. However, research and advancements in the field may have brought forth new methods or improvements since then.

Lexical analysis and syntactic analysis are fundamental stages in the process of language processing and understanding. They are key components in the compilation of programming languages, natural language processing (NLP), and other language-related tasks [5], [74]-[82].

Lexical analysis, also known as scanning or tokenization, is the initial phase of language processing. Its main objective is to break down the input text (source code or natural language sentence) into a sequence of tokens. Tokens are the smallest meaningful units of a language, such as words, keywords, operators, or punctuation marks.

The lexical analysis process involves steps such as Input Stream Directing, application of Lexical Rules, Tokenization, Token Streaming.

Morphosyntactic Analysis, also known as parsing, is the second phase of language processing and comes after lexical analysis. Its primary purpose is to determine the grammatical structure of the given input, checking if it conforms to the rules of a formal grammar for the language.

The syntactic analysis process involves the application of Grammar Rules, Parsing (namely, the token stream produced by the lexical analysis is analyzed based on the rules defined in the grammar and it is determined how the tokens fit together to form valid language constructs such as expressions, statements, or phrases), Syntax Tree, Error Handling.

The distinction between lexical analysis and syntactic analysis lies in the level of abstraction they operate at. Lexical analysis deals with individual tokens and their categorization, while syntactic analysis focuses on the arrangement and relationships of these tokens to form valid language constructs. Both processes are essential for understanding and processing languages, whether they are programming languages or natural languages.

Semantic analysis focuses on understanding the literal meaning of language, while pragmatic analysis deals with how language is used in context to convey meaning, intentions, and social interaction. Both semantic and pragmatic analyses are essential for a comprehensive understanding of language use in communication, and they are used most often following the lexical and morphosyntactic analysis.

More specifically, semantic analysis focuses on how words, phrases, sentences, and other linguistic units convey meaning and how these meanings are related to the real-world concepts they represent. The main objective of semantic analysis, beyond word/sentence meaning, is to discover lexical relations, and resolve ambiguities such as lexical or structural.

Pragmatic analysis focuses on the study of context by exploiting the principles and rules that dictate language use in specific communicative situations. It conveys inference, presupposition or implied meaning.

Phonetic Analysis is the study and analysis of speech sounds, focusing on the individual units of sound (phonemes) that make up spoken language. It involves examining the physical properties, articulatory characteristics, and acoustic features of speech sounds to understand how they are produced and perceived in different languages [83]-[87]. Phonetic analysis plays a crucial role in linguistic research, language teaching, speech therapy, and the development of speech recognition and synthesis technologies. It helps linguists and language researchers understand the intricate details of how sounds are produced and perceived, leading to a better understanding of the diversity and complexity of human languages.

Segmental and suprasegmental phonetic analysis are two fundamental components of phonetics that focus on different levels of speech sound analysis.

Segmental phonetic analysis deals with the individual speech sounds or segments, which are the discrete units of sound that form the basis of spoken language. These segments are also known as phonemes. In this analysis, the focus is on the articulatory properties, acoustic characteristics, and distinctive features of individual speech sounds.

Suprasegmental phonetic analysis focuses on the aspects of speech that go beyond individual segments and affect larger units of speech, such as syllables, words, phrases, and entire utterances. These suprasegmental features influence the rhythm, intonation, stress, and timing of speech, adding additional meaning and nuance to spoken language.

Key aspects of suprasegmental phonetic analysis include:
Stress and Intonation: analyzing how stress is placed on certain syllables or words within an utterance and how pitch variations (intonation) convey different types of information, such as questions, statements, or emotions;
Rhythm: Studying the timing patterns in speech, such as the duration of syllables and pauses between words, which can vary across languages and speech styles;
Prosody: Examining the overall melody or musicality of speech, which includes variations in pitch, rhythm, and stress patterns;
Sentence and Discourse Level Analysis: understanding how suprasegmental features can affect the meaning and interpretation of longer stretches of speech, such as sentences, paragraphs, or conversations.

Suprasegmental features are essential for conveying linguistic and paralinguistic information in speech. They help convey emotions, attitudes, and pragmatic meanings that go beyond the literal meanings of individual words and sentences.

Regarding Modeling and Classification, Automatic classification of speech and speaker profiles involves using machine learning and pattern recognition techniques to analyze and categorize speech signals based on various characteristics. These systems have numerous applications, including speaker identification, language recognition, emotion detection, and speech-based authentication [49], [88]-[99].

Key processes include:
As to Data Collection, a large dataset of speech samples that represent the target classes is often required. For speaker profiling, this dataset would include speech samples from different individuals. For speech classification tasks, the dataset may contain samples of different languages, emotions, or speech styles; regarding Feature Extraction, from each speech signal, relevant features need to be extracted to represent the speech characteristics effectively. Common features include Mel-frequency cepstral coefficients (MFCCs), pitch, energy, formants, and prosodic features;
as to Model Training, Machine learning algorithms are trained on the extracted features to create classification models. Popular techniques include Logistic Regression, Decision Trees, Random Forest, Support Vector Machines (SVM), Bayesian Models, Gaussian Mixture Models (GMM), Hidden Markov Models (HMM), neural networks -such as Convolutional Neural Networks or Recurrent Neural Networks-, LSTM, Boosting, and more recently, transformer-based models like BERT. Model training can be performed using supervised methods, unsupervised methods, or hybrid ones such as self-supervised and semi-supervised. In supervised methods, the models are trained using labeled data, where each sample is associated with the corresponding class or label, such as speaker identity, language, emotion. The models learn to map the extracted features to the target classes during this training phase. The unsupervised methods are used to cluster unlabeled datasets, such as similar speech profiles, without explicit labels. In semi-supervised methods, a small amount of labelled data is used to train a model, which is then used on an unlabeled dataset, and it is "tuned" by associating known labels and pseudo-labels (from the un-labelled data), In self-supervised methods, the model training actually also happens with data and labels, but the labels are generated by the model itself, and is recurrently used on new unlabeled data which by turn produce new labels, and so on. Self-supervised methods like BERT have been successfully applied to NLP;
as to Validation and Testing, the trained models are validated and tested using separate datasets to evaluate their performance. The evaluation metrics may include accuracy, precision, recall, F1-score, and confusion matrices;
an important issue is that building accurate and reliable speech and speaker classification systems often requires large and diverse datasets, careful feature engineering, and well-optimized machine learning models. Additionally, the performance of these systems may vary depending on the quality and variability of the speech data and the complexity of the classification task.

Automated assessment systems for cognitive tasks use technology and algorithms to evaluate an individual's performance on various cognitive tasks. These systems are designed to analyze and measure cognitive abilities such as memory, attention, problem-solving, reasoning, and other mental processes. They find applications in various fields, including education, healthcare, research, and cognitive training [46], [82], [100]-[107]. Key components commonly used in automated cognitive performance assessment are Data Collection, Machine Learning Algorithms, Normative Databases: as to Data Collection, the system collects data during the individual's interaction with the cognitive tasks. This data can include response times, accuracy, keystrokes, eye movements notably using eye-tracking methodology..

As to Machine Learning Algorithms, Automated cognitive assessment systems often use machine learning algorithms to process and analyze the collected data. These algorithms are able to identify patterns, trends, and performance metrics that are indicative of cognitive abilities. They may be pre-trained on large datasets to recognize cognitive patterns or may adapt and improve its assessment over time as it collects more data;
as to Normative Databases, optionally, some systems use normative databases to compare an individual's performance against a reference group of individuals of similar demographics, such as age, education level, to establish a baseline for cognitive functioning. This comparison helps identify deviations from the norm and potential cognitive deficits.

Automated cognitive assessment systems have the potential to complement traditional cognitive assessments, providing efficient and objective measurements of cognitive performance. However, it is essential to validate these systems against established standardized methods and procedures of assessment, often performed by human judges.

### Prior Art

KR102336015B1 for a "video-based language disorder analysis" discloses a video-based language disorder analysis system, method, and program with the purpose to analyze speech disorders by processing video and audio of patients, focusing on speech fluency, speed, and completeness of sentences. Technology consists of a server-based system using speech recognition and sentence analysis; whereas the input data consists of video and audio recordings from clients, which are processed on a server. The speech features analyzed are speech speed, sentence length, non-fluency -both normal and pathological-, morphemes and reaction time between speakers. The processing methodology is as follows: the client records the video/audio, sends it to a server where speech recognition and sentence completeness are analyzed. The system also detects fluency issues -normal versus pathological-.

The core analysis steps consist of detecting speech items and speaker identity, performing speech recognition to convert audio into text, analyzing fluency, speech speed, morphemes, and sentence completeness, detecting non-fluency in speech and categorizing it as "normal" or "pathological". Further features consist of client-server model with real-time video analysis; with focus on sentence completeness and fluency detection; wherein non-fluency is divided into normal and pathological types, helping early diagnosis.

However, no use of machine learning is directly mentioned; it uses server-side processing for fluency and sentence analysis. As output, the client receives an analysis of speech fluency, speed, completeness, and non-fluency types -normal versus pathological-.The system thus provides a video-based analysis thus allowing for early detection of speech disorders, whereas distinguishing between normal and pathological speech is helped by a detailed non-fluency categorization This system has certain limitations though among which heavily relying on server-side processing, which may be considered a point of weakness.

US11145321 for "Aphasia Diagnosis via Machine Learning" relates to methods and systems for using machine learning to diagnose aphasia with the purpose of diagnosing various types of aphasia using machine learning on spontaneous speech recordings.

The technology involves stacked machine learning classifiers for fluency and comprehension assessment. The Input Data consists of audio or video recordings of spontaneous speech, transcribed into text and analyzed. The Speech Features Analyzed are words per minute, unique words, verbs/nouns count, repeated words, and spectrographic components to classify different aphasia types.

Processing Methodology uses a two-step classification including Speech quantification generating a fluency score, and a comprehension analysis generating a comprehension score, wherein both are used to classify aphasia types such as Broca's, Wernicke's.

The Core Analysis steps comprise speech-to-text processing; quantifying speech using words per minute, verbs/nouns, repetitions; assessing comprehension by comparing fluency and speech patterns; Stacking classifiers to generate a diagnosis based on fluency and comprehension scores, wherein the Stacked classifiers improve diagnosis accuracy by sequentially analyzing fluency and comprehension. There is provided a Confidence-based classification of different aphasia types, whereas it can assess various aphasia subtypes, improving diagnostic granularity.

The use of Machine Learning involves stacked machine learning classifiers for aphasia classification, which are trained using tagged speech data. The output produces aphasia classification along with fluency and comprehension scores suggesting therapies. Aphasia diagnosis backlog is reduced by allowing non-specialists to administer tests; diagnostic processes are standardized reducing inconsistencies across clinicians; multiple aphasia types can be classified with confidence scores. However, it is primarily focused on aphasia diagnosis, which is limited regarding the applicability range of the methods and systems disclosed therein, thus constituting a lack when it comes to other speech disorders or diseases.

US20210121124A1 for "Speech Pathology Diagnosis with ML" relates to a system and method for diagnosing and treating speech pathologies with the purpose to detect and classify pathological speech using a deep neural network, without requiring large sets of tagged pathological data. Technology involves deep learning techniques using Recurrent Neural Networks (RNN) with novelty detection algorithms. The Input Data consists of audio recordings of speech that are compared to normal speech samples using a trained classifier. The Speech Features Analyzed are prosody, intelligibility, fluency, vocabulary, accent, emotion, jitter, shimmer and more detailed metrics like comprehension, planning and grammar.

Processing Methodology is as follows: a pre-trained speech classifier based on non-pathological speech uses novelty detection to identify abnormal speech. If speech is tagged as abnormal, a secondary classifier determines specific pathologies like stuttering, aphasia or Parkinson's.

The Core Analysis Steps consist of using novelty detection to compare new speech with trained normal speech; detecting abnormal patterns and classifying them as pathological; sub-classifying the speech based on identified pathologies, such as stuttering, aphasia, and articulation problems. Further features consist of using a pre-trained network on normal speech reducing the need for vast pathological datasets; identifying specific speech disorders by a secondary classifier, supporting more precise diagnosis, such as Parkinson's, articulation issues; wherein a language agnostic classifier focuses on speech prosody and quality. The use of Machine Learning involves deep neural networks (DNN) for speech pathology diagnosis, with novelty detection algorithms to differentiate between normal and pathological speech.

As output, speech is classified as normal or pathological, with specific pathologies identified, such as stuttering, aphasia, articulation issues, based on machine learning models.

This system thus provides a diagnosis without the need of large datasets of tagged pathological speech; it can be implemented in multiple languages; and it is scalable to detect various pathologies without the need for constant retraining.

However, this system has limitations in that it requires initial training on normal speech, whereas pathology detection is dependent on novelty detection which might struggle with borderline cases, which thus appears to be lack of this system for not fully covering the latter cases.

CN1 03505183B relates to a multidimensional speech disorder measurement system for the diagnosis and adaptation of rehabilitation programs. It is based on multidimensional modeling of speech in real time and includes -a data collection device such as a microphone, electroacoustic transducer, laryngoscope and a mouth-nose separation collector; -a computing unit for calculating and analyzing speech parameters such as frequency, intensity, time of maximum vocalization, vocal cord vibration and nasal sound flow ; -an evaluation unit that compares the calculated values with standard reference ranges to identify and classify the type of disorder ; -a rehabilitation monitoring unit which compares successive measurements to assess the progress of the treatment. Its specific difference resides in the Operational step of the method proposed below.

In view of the preceding, it is represented that existing systems for speech pathology diagnosis often rely on laborious manual evaluation or require vast datasets of tagged pathological speech. This results in inefficiencies, limited scalability, and poor adaptability across languages. Even when computational tools are used, they remain restricted to basic acoustic parameter extraction or static reference comparisons, without adaptive profiling or feedback. The only other options currently available to clinicians are costly face-to-face evaluations or fragmented digital assessments, both of which fail to ensure accuracy, repeatability, and sustained diagnostic reliability. This complex process leads to ineffective outcomes and delays in rehabilitation, which constitutes a drawback.

It is highly desirable to streamline and automate speech pathology assessment by ensuring that speech data is not only collected but also processed, profiled, and scored against predetermined task targets, while continuously updating subject profiles with new data and feedback. Current systems require large pathological datasets and manual interpretation, which is costly and inefficient, and existing AI tools remain limited to narrow acoustic analysis without adaptive feedback or multimodal integration.

### Aim of the invention

The aim of the invention consists in remedying the drawbacks referred to above and developing a solution to the above-mentioned problems regarding speech disorders and limitations by remedying to the shortcomings of known systems and methods above notably including a collective and integrated methodological and functional/operational flow and a system's synthesis for an integrated, intelligent, automated, software/hardware system which performs in a collaborative manner, both in terms of interconnections between modules or other external systems and/or in cooperation with human operators, based on a set of general operations.

The purpose of the invention is to collect, process, analyze a subject's multimodal reaction/response data with the aim to judge the correctness and profile of the latter's speech responses, in various types of naming tasks, under conditions of w/ & w/o brain stimulation, and adaptively individualize the naming task procedure's parameters and execution, as well as optimally control any brain stimulation's paradigm and parameters that are involved in the concurrent conduction of a Naming Task and Brain Stimulation.

### Summary of the invention

Accordingly, the method proposed according to the invention involves the following general operations designated as Op1 to Op5 set out hereafter:

| | |
|---|---|
| Op1. | Collection of subject responses, along with a variety of additional data; |
| Op2. | Removal of any unwanted signals that may be mixed with the subject speech; |
| Op3. | Performance of phonetical, phonological, lexical, semantic, and pragmatic NLP along/or after the speech's content recognition; |
| Op4. | Performance of intelligent and adaptive coordination of execution flow and selection of the task/paradigm's presentation and interventional parameters; |
| Op5. | Building of a system that is structured to be trainable and supervisable, esp. recurrently accumulates, analyses and makes decisions upon its inputs and outputs, and thus constitutes an intelligent system. |

Thanks to the method of the present invention, there is performed automatically and fast with minimal human judge or operator effort.

According to the present invention, there is thus proposed a structured solution for the Acquisition and Automated Analysis, Assessment and Scoring of Subjects' speech, under a variety of paradigms/tasks, such as those mentioned above notably including Frameworks of complex tasks related to cognition and speech production, Rehabilitation for Speech-Language Disorders, and interventional setups, Brain Stimulation approaches in cognitive and motor processes related to speech, in the form of a computer and network suite, for use in clinical/non-clinical research, rehabilitation, training and assessment of speech, which method is remarkable in that it comprises the following steps : a data & response step a, wherein subject's multimodal raw data related to task and/or stimulation/testing regarding speech-related responses are collected together with Electronic Health Record (EHR) data, and task/paradigm/stimulation data, wherein said data are used in subsequent steps to start with a speech extraction step b, wherein subject's speech/voice signals are detected, subsequently identified, further discriminated and isolated from unrelated activity or signals, notably noises, yielding a clean signal that is fed forward to a next step: an utterance processing step c, wherein the extracted speech signal is processed and analyzed, esp. by means of a NLP process, generating a detailed utterance structure.

In a further functional step d, two functions are performed in parallel: firstly an assessment and indices function, wherein said detailed utterance description, content, structure, events are evaluated and quantified against a set of predetermined task/paradigm targets, yielding a characterization and scoring for a specific subject's performance,
and secondly a modeling & profiling function, wherein the same detailed utterance description, content, structure, events, are fed to a modeling, classification, and profiling module that generates profiles of utterance specific, subject specific and population specific performance as well as profiles of speech characteristics, in conjunction to a set of parameters, wherein this profiling is used both as an autonomous output and as feedback to the above-mentioned utterance processing step for enhancing accuracy and speed of the process.

In a process coordination step e, the results of the analysis and profiling are fed back to a processing unit which evaluates in real time the optimum parameters as target and conditions for the following steps of the subject's testing for maximizing the informational content of the profiles at the individual subject level and/or at population level;
it additionally comprises a database storage step f wherein, at all steps, data, inputs and outputs are exchanged with a local and/or remote database storage unit.

It is fully trainable and supervisable through a training & supervision step o at all steps, esp. wherein it uses decision making, thereby accumulating knowledge.

Its specific difference lies in the Operational step of the method which is not disclosed in document CN103505183B referred to above.

A particular feature of the method of the invention is to extract, quantify, evaluate and assess the subject's speech and/or its characteristics, such as accuracy, quantification of diversions or adherence or conformance to the demanded task, namely errors, semantics, lexical, pragmatics, phonetics, timings, sequential characteristics, of the response, in relation to the offered visual, auditory, olfactory, tactile, vibratory, motional template and demanded task.

A further feature of the present invention is to perform classification, clustering and modeling of the content and signal characteristics of the sequences of events in the subject responses, producing appropriate profiles of utterance and speaker.

A still further feature of the invention, said analysis above is augmented by appropriate feedback from previous utterance's/speaker's profiles, which are formed by accumulation of previous analyses or beforehand knowledge.

Another further feature of the invention is to maximize and optimize the acquired subject or population profile information through the adaptive coordination of the process.

According to a *main* embodiment of the invention, there is proposed a Computer-implemented method of collection and automated analysis, evaluation and scoring of speech of a subject, executed on a computer system comprising processor, memory, input/output units and database, within a set of tasks
- tests related to cognitive function and speech production, rehabilitation of speech-language disorders and intervention devices such as brain stimulation devices (TMS/DCS), intended for rehabilitation, training and evaluation of speech, which is characterized in that it includes the following steps executed through the above computer system:
- a data and response step, where through sensors and computational interfaces structured multimodal primary data of the subject are collected which relate to task-process-work and/or stimulation/examination regarding responses related to speech and especially together with structured Electronic Health Record (EHR) data and structured data of test-process-work/paradigm/stimulation, where said data are used in the subsequent steps,
- a speech extraction step, implemented by a signal processing unit, where the speech/voice signals of the subject are detected, then recognized, further distinguished and isolated from irrelevant signals, mainly noise, other speech, etc., creating a digital clean signal which is further fed into a next step;
- a further speech processing stage, implemented by a computational processing unit and speech-recognition software, where the extracted speech signal is processed and analyzed, producing and extracting a detailed utterance structure, including timings, phonetic, lexical, semantic and pragmatic features;
- a further functional step, where two functions are executed in parallel consisting of a first evaluation and indices function, where the said detailed utterance structure and the events are evaluated and quantified against a set of predefined targets of task-process/paradigm, providing characterization and scoring for the performance of a specific subject, and a second modeling and profiling function, where the same detailed speech structure and events are simultaneously fed into a modeling-classification-profiling block constituting a machine learning (ML) sub-module implemented in software on the processor of the system, which applies classifiers or regression/prediction models based on techniques such as Gradient Boosting Machines (XGBoost), Random Forests or Support Vector Machines (SVM), so as to identify non-linear patterns and create performance profiles of specific utterance, specific subject and specific population, as well as speech characteristic profiles, in combination with a set of parameters, where this profile is used as an autonomous output and as feedback to the above speech processing step to improve the accuracy and speed of the process, as well as software function, structure and implementation whereby the ML model is trained and readjusted in real time, receiving data from the database of the system, while its parameters and hyper-parameters are automatically computed and optimized;
- a process coordination step (150), where the results of analysis and profile are fed back for processing, implemented by a processing and control unit in the form of computer software which evaluates in real time the produced profiles and adjusts the parameters and target conditions for the following steps, including: (i) the conditions of stimulus presentation -esp. time, type, of images/sounds/other stimuli, and (ii) the settings of interventional brain stimulation devices, -notably TMS, DCS,-, so that a technical result is achieved consisting of real adaptation of the physical parameters of those devices through the outputs of the computing system, esp. for maximizing the informational content of the profiles at individual subject or population level;
- an additional database storage step where all data, inputs and outputs of the previous stages are exchanged, in all steps, with a local and/or remote database storage unit of the system and are used for feedback re-training of the ML model, ensuring continuous optimization of prediction accuracy and adaptive settings; and
- a training and supervision step through a computing training unit in which the system is fully trainable and supervisable in all the preceding steps, through remote or local computer interface or cloud infrastructure.

The method proposed by the invention essentially provides a computer-implemented method executed by a processor for the acquisition, assessment and scoring of a subject's speech under a set of tasks related to cognition and speech production in speech-related cognitive and motor processes, wherein the method comprises the following steps:
a data and response step, for collecting the subject's multimodal raw data related to task, wherein said raw data comprises stimulation/testing data regarding speech-related responses and Electronic Health Record (EHR) data;
a speech extraction step, for extracting the subject's speech/voice signals from the collected raw data by detecting, identifying, discriminating and isolating the subject's speech from unrelated signals, and generating a clean signal;
an utterance processing step for analyzing the extracted speech signal to generate a detailed utterance structure,
wherein said detailed utterance structure is evaluated and quantified, by a first assessment and indices function, against a set of predetermined task/paradigm targets for yielding a characterization and scoring for the subject's performance, and simultaneously modeled, by a modeling and profiling module for generating profiles of utterance specific, subject specific, population specific performance, and speech characteristics, based on a set of parameters,
wherein the generated profiles is used both as an autonomous output and as feedback to the utterance processing step for enhancing accuracy and speed of the process, and the result of analysis and the generated profiles are fed back to a processing unit which evaluates, in real-time, the predetermined targets and the set of parameters for maximizing the informational content of the generated profiles at the subject level and/or at the population level,
a database storage step for storing the data, inputs and outputs, in a local and/or remote database storage unit; and
wherein the method further includes a training and supervision step (100), to fully train and supervise all the steps of the method.

The technical modules and coordinated operations required for acquisition, extraction, processing, assessment, profiling, coordination, storage, and training/supervision are thus provided by the invention. Regarding CN103505183B relating to a multidimensional speech disorder measurement system, while it discloses data collection devices, a computing unit for analyzing speech parameters, and an evaluation unit comparing values with reference ranges, the specific differences from the invention lie in the operational steps of the proposed method, which include multimodal acquisition, clean signal extraction, structured utterance generation, parallel assessment and profiling, and real-time feedback coordination.

According to a preferred embodiment of the invention, the method for an Automated, Adaptive and Intelligent Profiling of Speech Responses in Mapping, Diagnosis, Treatment and Rehabilitation, is based on the following steps:
Step 1 of Data & Responses, wherein Subject's multimodal raw data related to task and/or stimulation/testing regarding speech-related responses, -such as speech/voice recordings, video recordings of facial/motional/positional/gestural state, vibratory state, neuro-muscular activity-, together with Electronic Health Record (EHR) data, and task/paradigm/stimulation data are collected and used in subsequent steps.
Step 2 of Speech Extraction, wherein_the subject speech/voice signals are detected, identified, discriminated and isolated from any unrelated activity, -such as noises, artifacts-, and fed forward to the next step.
Step 3 of Utterance Processing, wherein the extracted speech signal is processed and analyzed using NLP methods and techniques and detailed utterance structure is obtained, in terms of transcribed content, timings, phonetics, identification of singular events, such as hesitations & elongations.
Step 4 wherein two functions are performed in parallel:
   firstly Assessment and Indices, wherein the detailed utterance description, content, structure and events, are evaluated and quantified against the predetermined task/paradigm targets/objectives, offering a characterization and scoring for the specific subject's performance;
   secondly Modeling & Profiling, wherein the same detailed utterance description, content, structure, events, are fed to a modeling, classification and profiling system that generates profiles of utterance specific, subject specific and population specific performance as well as profiles of speech characteristics, in conjunction to the various health, demographics, task/paradigm and stimulation parameters. This profiling is used both as an autonomous output and as feedback to the Utterance Processing step aiming to the improvement of accuracy and speed;
Step 5 of Process Coordination, esp. wherein the results of the analysis and profiling are fed back to a processing unit which appropriately evaluates, esp. in real-time, the optimum parameters and conditions for the following steps of the subject's testing, in order to maximize the informational content of the profiles both at the individual subject and/or at population levels. More particularly, the processing unit decides the on the content characteristics, presentation levels, timings, etc. regarding the sensory templates offered to the subject -such as pictures, sounds- as well as any parameters regarding possible external interventions to the subject's state -such as TMS, DCS-, or any other type of stimulation/disturbance such as intensities, patterns, timings and spatial/volumetric distributions.

The method employs extensive Machine Learning ML techniques at virtually all steps. It is fully trainable and supervisable in a Training & Supervision step at all steps, and it makes extensive use of decision making thus resulting in the ability to accumulate knowledge thereby constituting a smart resp. intelligent system. Additionally, at all steps, data, inputs and outputs are exchanged with a complete local, remote or mixed database storage system referred to as Database Storage.

The speech features analyzed with the method of the invention are morphological. Analysis, concurrent lexical, syntactic, semantic and pragmatic analysis and annotation of utterance, as well as Discrete behavioral sequences of speech and language units such as phonemes, words, syllables, phrases (complete or incomplete) various phonetic events such as phonemes /word parts elongations; and also various segmental/suprasegmental speech units/events, the Recognition of realized target word/phrase.

According to a further embodiment of the method of the invention, said tasks comprise Frameworks of complex tasks related to cognition and speech production, Rehabilitation for Speech-Language Disorders- and interventional setups, wherein in said data & responses step, these subjects' multimodal raw data are collected which comprise speech/voice recordings, video recordings of facial/motional/positional/gestural state, vibratory state, neuro-muscular activity, and/or in said utterance processing step, said detailed utterance description content, structure is generated therewith, notably in terms of transcribed content, timings, phonetics, identification of singular events such as hesitations, elongations; wherein said set of parameters comprises health parameters.

According to a still further embodiment of the method of the invention, sensory templates comprising pictures, sounds are presented to the subject, as well as further parameters regarding external interventions to the subject's state, such as Transcranial Magnetic Stimulation (TMS), DCS, or a stimulation/disturbance such as intensities, patterns, timings, spatial/volumetric distributions, wherein said processing unit decides on the content characteristics, presentation levels, timings, regarding said subject's sensory templates.

According to a yet further embodiment of the method of the invention, Speech Responses are analyzed and profiled in a Picture Naming Task under said TMS stimulation wherein said task is defined as a picture naming task, the subject's ability to name pictures under said TMS stimulation is assessed for purposes of brain mapping of speech production structures, wherein two phases ϕ₁, ϕ₂ are involved, wherein
- in a first phase ϕ₁ not including said TMS stimulation, a form of subject's baseline performance characteristics is acquired by presenting pictures to the subject, whereas the subject is asked to name them, and the responses are recorded and analyzed, thereby providing with reference performance values and information on an appropriate parameterization of the process, which yields results to be used in the following phase ϕ₂, and
- a second phase ϕ₂ constitutes the main brain mapping paradigm using said TMS stimulation, which is performed after the specification of the paradigm's parameters based on the results of said first phase ϕ₁.

According to a more specific embodiment of the invention, in the first phase ϕ₁, pictures associated to abnormal subject's naming responses are identified, after which said pictures are removed from the presentation material of the subsequent actual mapping forming sub-step (a), and the interpicture interval (IPI) and the picture presentation time (PPT) are adjusted whereby they are fitted to the subject's performance ability forming sub-step (b), particularly wherein the automated process of cleaning recorded responses from said undesired intruding signals, and speech analysis and recognition provide the desired information for said sub-steps (a) and (b), thereby yielding an objectified identification of abnormal responses and a customized algorithmic adjustment of said interpicture interval (IPI) and the picture presentation time (PPT). More particularly response characteristics are registered therein at each trial, wherein the collection of the values for each said characteristic forms the distribution of the characteristic at normal response, thereby serving as template for determining responses to be either normal or abnormal, the latter being induced by said TMS stimulation in the subsequent second phase ϕ₂, whereas more specific classifications of abnormal response are also defined in said second phase ϕ₂, wherein the normal uttering/speech characteristic distributions are subject-specific and adapted to the designed interpicture interval (IPI) and the picture presentation time referred to as PPT.

According to a further specific embodiment of the invention, in said second phase ϕ₂, the remaining presentation material not being discarded after said first phase ϕ₁, is presented to the subject, while said TMS stimulation is performed over a section of the cortical region in the perisylvian area, with a dense spatial sampling perilesionally, esp. in synchrony with the appearance of pictures, particularly wherein picture-to-TMS trigger interval (PTI) = 0 ms,
More particularly, a range comprised between 40 to 80 sites in each brain hemisphere is stimulated at a sequence, esp. three nonconsecutive times each resulting in a total of 120 to 240 stimulations per brain hemisphere, thereby including both the lesion-harboring brain hemisphere as well as the contralateral one, after which lesion-induced plasticity phenomena are identified.

The response to each said stimulus is classified as either normal or abnormal, whereas in the latter case it is further classified as no response designated as speech arrest or aphasic anomia; resp. performance error including dysarthria or speech apraxia; hesitation, circumlocution, paraphasias - semantic or phonological- and neologisms.

According to a particular embodiment of the invention, the final stimulation paradigm employed in said second phase ϕ₂ is adaptive, wherein the full parametrization of the stimulation paradigm, notably of the procedure, comprising stimulus intensity and frequency, number of stimuli, the abovementioned IPI, PPT and PTI, esp. under use of said first phase ϕ₁, based on the speed and accuracy of the automated extraction of results, yields an objective identification of abnormal responses, thereby allowing an accurate, customized parametrization of the full set of stimulation parameters. According to a more particular embodiment of the invention, through a training period of said first phase ϕ₁ and said second phase ϕ₂, threshold values defining distribution tails of each normal uttering/speech characteristic are set, including at very low significance level, wherein said threshold values determine the deviation from normal of each speech characteristic and classify in terms of each characteristic the response as normal or abnormal.

In particular, the subject's responses, esp. video-recorded, from said second phase ϕ₂ are analyzed and each response is again classified as normal or abnormal, wherein in the latter case it is further classified as "no response", "performance error", hesitation, circumlocution, paraphasias and neologisms, wherein every brain stimulation site where an error is identified on at least two nonconsecutive times is regarded as nr TMS-positive and indicates a functional, language-related brain tissue.

According to an even more particular embodiment of the invention, the identification of abnormal responses is performed in real-time, thereby generating an objectified identification of abnormal responses and a detailed subject's utterance profile, thereby subsequently yielding the construction of a subject's language network brain map. In particular, additionally, fusion schemes are realized based on outcomes of this automated & adaptive method -esp. the decision whether each uttering/speech characteristic is normal or abnormal- at each of three repetitions, enabling to decide for establishing the overall response as normal or abnormal, as well as to give a score of abnormality based on the decision on each of the specific abnormality types, among the abovementioned "no response", "performance error", hesitation, circumlocution, paraphasias and neologisms.

The processing method is summarized as follows:
Within any type of Naming Task, multimodal data -notably speech, gestural, myographic, haptic- are collected and complement acoustic scene analysis for speech extraction, removal of unwanted signals, tagging and various NLP procedures for the identification of various markers of divergence between responses w/ and/or w/o Brain stimulation;
ML models of speaker / utterance's profiles are generated by exploitation of various accessory data, notably demographics, HER, stimulation parameters, naming task's templates. These models are subsequently employed to adaptively fine tune the naming tasks' procedure, content and parameters, and the Brain Stimulation's parameterization for efficient and individualized depiction of brain stimulation's effects upon speech abilities and subsequent identification of brain circuits;
All data, decision making for procedural parameters, speaker profiles, and stimulation are accumulated in a Database; and can be subjected to an external judge's review/assessment and subsequent adaptation of the employed algorithmic targets.

The core analysis steps consist of employing extensive multimodal signal processing for Acoustic Scene Analysis, detection and isolation of speech responses and extensive NLP for identification and tagging of speech and/or non-speech response events and builds statistical speaker/utterances' models by employing accessory data.

ML techniques are employed with the above in order to adaptively fine-tune all kinds of parameters and execution steps of the Naming Task and/or Brain Stimulation procedures.

The distinguishing features notably include the Exploitation of multimodal data streams and additional accessory data. In addition, the speech responses' analysis is used both for speech/speaker profiling and ML-based tuning of the Naming Tasks and Brain Stimulation procedures.

All processes are realized in real-time, and the adaptive character of the ML-based tuning of the Naming Tasks and Brain Stimulation manifests itself through the concurrent/real-time re-adjustment of processes/parameters of interest with the procedures' execution.

External judge's revision allows for expert adjustment of algorithms and enhanced reliability of the method and system of the invention.

ML is inherently employed in most of the processing and decision-making modules, such as scene analysis, multimodal data processing and analysis, NLP, adaptation of tasks and brain stimulation parameters/protocols and expert revision.

The output consists of Statistical models of speech/speaker's profiles under various conditions, such as demographics, EHR, stimulation parameters and naming task's templates.

It generates adaptive adjustments of naming tasks' and brain stimulation's parameters.

The present invention also relates to a system for an Automated, Adaptive and Intelligent Profiling of Speech Responses in Mapping, Diagnosis, Treatment and Rehabilitation, wherein the technology involved is implemented by means of a plurality of functional modules represented by blocks which are selectively interconnected, comprising the following : according to a basic embodiment of the system of the invention, it is specifically designed for carrying out the method as defined above, esp. through an integrated, automated, software/hardware performing in a collaborative manner in terms of interconnections between modules and/or in cooperation with human operators. Accordingly, the system is remarkable in that it comprises a data means unit for the acquisition of multimodal subject's responses as well as the collection of subject's data, which is interactively interconnected with a speech extraction means unit for subject's speech and voice discrimination and isolation from unrelated activity or signals, which is further connected to an utterance processing means unit for utterance analysis and recognition, which in turn is further connected to a functional means unit consisting of an assessment and indices means unit for utterance assessment according to paradigm tasks and estimation of indices as first functional unit, on the one hand, and a modelling and profiling means unit for utterance and subject's modelling and profiling as second functional unit, on the other hand, which is connected to said first functional unit. The latter second functional unit is connected retroactively with the above-mentioned utterance processing means unit.

It further comprises a process coordination means unit that is interactively interconnected with said functional means unit and the above-mentioned data and response means unit including with a retroactive connection thereto. A database storage means unit is further provided that is connected to said data means unit, said speech extraction means unit, said utterance processing means unit and said functional means unit respectively. A training and supervision means unit is further provided which is interactively interconnected with said data and response means unit, said speech extraction means unit, said utterance processing means unit and said functional means unit resp.

The corresponding data processing system implementing the method is thus defined, notably through detailing the multimodal data collection, utterance processing, profiling, and feedback mechanisms.

According to a further embodiment of the system of the invention, it is designed for the Acquisition and Automated Analysis, Assessment and Scoring of Subjects' speech, under a set of tasks comprising Frameworks of complex tasks related to cognition and speech production, Rehabilitation for Speech-Language Disorders- and interventional setups, in the form of a computer and network notably intended for rehabilitation, training and assessment of speech, wherein it comprises a set of operating means for a collective and integrated methodological and functional/operational flow of data means and a system's synthesis means for an integrated, intelligent, automated, software/hardware system which performs interactively in terms of interconnections between modules and/or in cooperation with operators, the said operations means comprising:
- Collection means of subject's responses, along with a set of additional data means,
- Removal means of undesired signals from the subject's speech,
- Means for the performance of phonetical, phonological, lexical, semantic, and pragmatic NLP along or after the speech's content recognition,
- Means for the Performance of intelligent and adaptive coordination of execution flow and selection of the task presentation and interventional parameters,
- Building means for building a system structure that is trainable and supervisable, which recurrently accumulates, analyses and makes decisions upon reception of an inputs resp. outputs, esp. thereby generating a so-called smart system.

According to a preferred embodiment of the system of the invention, it has a plurality of input ports comprising a primary main input stream constituted by raw signal data collected from the subject, task specific data/metadata, and undesirable signals, as well as an accessory input stream constituted by additional data apart from said primary input stream, as well as an input module of task descriptors and targets predefining quantitative and qualitative indices of interest as well as the targets of a specific experimental paradigm.

It further comprises analysis means performing an analysis of a subject's scene, esp. identification of events that are captured by a set of sensors and whose signals are contained in said primary input stream, wherein the analysis of events regarding the subject's responses and/or an additional external event taking place in the subject's scene is performed in said analysis means by virtue whereof eventful source of information contained in said primary input stream is identified and classified, by means whereof said events are discretized, whereas the said data are fed to the next module consisting of filtration means, wherein a discrimination is performed in said filtration means between target-of-interest referred to as TOI responses/reactions from the subject and other eventful signals which are not of interest constituting unwanted signals, which are eliminated thereby isolating the TOI for further analysis.

Said unwanted signals are eliminated, thus yielding a clarified TOI that contains a subject's isolated and purified utterance speech for the subsequent analyses.

The remaining cleaned parts of the utterance are detected, identified, classified and segregated in detection means in terms of phonemic/phonological units, wherein a logical classification of content as events is performed, that outputs a deliverable that is readily available, particularly as a table with timings and nature of the utterance.

The speech units -of which silence is excluded- identified in said detection means are transcribed into phonetic symbols and strings in transcription means, wherein morphological analysis, concurrent lexical, syntactic, semantic, and pragmatic analysis and annotation of the whole subject's utterance is performed in a main processing unit, wherein it recognizes discrete behavioral sequences of speech and language units and recognizes the target word/phrase through syntactic/pragmatic analysis.

Said main processing unit respectively produces probabilistic hierarchies of results that contain the identifications and marks of the said parts of the utterance that correspond to the subject's intentioned response to the task, wherein sequenced, layered, object oriented ontological models of the said utterance are built by said building means, thereby generating deliverables that are readily readable in a lecture format, particularly by tables, for use in a profile processor means. A phonetic analysis of each part of the utterance is performed in said phonetic analyzer means, by identifying and quantifying any phonetic parameters of interest, wherein data, inputs and outputs are stored in a database means -local or remote or mixed- and exchanged with above-mentioned means.

According to a particular embodiment of the system of the invention, it has additional processor means taking inputs from said processor unit, said phonetic analyzer means and task targets and quantifies the various parameters of interest (POI) as well as the degree of conformance in terms of accuracy or repeatability, namely types and numbers of divergences/errors from the specified task targets. Process coordinator means adaptively coordinate the task's/paradigm's presentation and/or intervention parameters, according to specific statistical or informational rules/targets, particularly wherein said process coordinator means suggests the values of the stimulation parameters and the parameters or content of the presented templates to subject, such as sounds, pictures, resp. or even directly controls the software/hardware means performing the stimulation and template presentation.

According to a more particular embodiment of the system of the invention, it outputs a table with timings and nature of the utterance, particularly wherein additionally, prior statistical knowledge of the subject's speech profile is also incorporated as feedback from phonetic analyzer means. More particularly in a similar probabilistic hierarchy, said processor unit outputs a complete table of fully time marked, transcribed, lexically, syntactically, semantically and pragmatically annotated written content of the subject's utterance, preferably wherein said processor unit operates on the audio of the speech signal, as well as in a combined stream of multimodal signals, such as audio-visual esp. audio together with video recording of gestural, facial, motional states, vibrational and neuro-muscular.

According to a still more particular embodiment of the system of the invention, the analysis in modules is upgraded, esp. wherein the registered and exchanged information with the database modules -in the form of records and fields or as object-oriented structures- includes information collected from said input modules I2, I3, and generated by said modules K, E, F such as subject's demographics, session place/time/duration details, paradigm/task parameters/components/ templates, interventional parameters/components/templates, wherein the profiles are created by combining and aggregating any of the above information types.

According to a yet more particular embodiment of the invention, profiles are continuously updated with the accumulation of new data and analysis results from said modules D, E, F, such as in each presentation during a repetitive naming task, and incorporating a decision-making step such as maximum likelihood selection, the module's output is fed back to modules D, esp. D1, D2, D3 for further improving their statistical performance, in terms of prior statistical knowledge of the subject's speech profiles.

The advantages achieved by the invention are notably operating in Real-time, with an automated process for the detection of brain networks; it is intelligent, adaptive and self-evolving algorithms and methodologies employing continuous accumulation of data. It is used for guidance of various Brain Stimulation schemas, appropriate for individualized use (prescription medicine), as well as clinical (laboratory and/or peri-operative) and non-clinical purpose, and it is applicable to various speech conditions (normal or any type of pathology).

Thanks to the system of the invention, there is performed automatically and fast with minimal human judge/operator effort.

The present invention also relates to the use of the method as defined above for the Acquisition and Automated Analysis, Assessment and Scoring of Subjects' speech, resp. of said system as defined above, under a variety of said paradigms/tasks in clinical/non-clinical research, rehabilitation, training and assessment of speech, wherein said use is performed by implementation in a computing system or software platform which interacts with all devices of acquisition, presentation or stimulation and exhibits and creates the appropriate technical effect accordingly.

In summary, there is thus provided thanks to the present invention,
- an explicit technical implementation background notably including computational means - hardware/software;
- with defining a clear technical result notably including adjustment of parameters of real devices;
- aligning with the criteria of Computer Implemented Inventions.

The present invention is executed exclusively through a computing system -local or cloud computing-which includes processors, memories, database, and input/output interfaces for the processing of real physical speech and sensor signals, structured data and the derivatives/results of the intermediate processing stages. The system affects real stimulus presentation and stimulation devices -notably including TMS, DCS,- causing a technical result in the form of actual adjustment of their parameters. The above functions, through appropriate implementations of their software, have the ability of external supervision, training and continuous adaptation of their parameters and structure. Consequently, the invention involves a technical character whereas the invention is not a mere mathematical method or organizational concept. The system and method appear to provide a concrete technological solution to a longstanding challenge in speech rehabilitation and pathology assessment: enabling automated acquisition, analysis, and scoring of speech responses under structured tasks related to cognition and motor processes, using multimodal data and machine learning infrastructure. Technical mechanisms are incorporated that process multimodal raw data, isolate clean speech signals, generate structured utterance data, quantify utterances against task/paradigm targets, and build statistical profiles at utterance, subject, and population levels. These profiles are generated at the utterance, subject, and population levels and are used both as diagnostic outputs and as feedback to the utterance processing step. By coordinating the analysis and profiling with the processing unit in real time, the system evaluates target parameters and conditions for subsequent steps, thereby ensuring alignment and integrity of diagnostic outcomes. Data is exchanged and stored across modules in local or remote databases.

The invention also demonstrates sustained adaptability through its reliance on multimodal inputs (audio, visual, neuromuscular signals) and high-intent interactions in profiling. By giving weight to recent data, incorporating diagnostic verification, and cross-validating across modules, the system ensures that speech assessment remains continuously relevant and accurate. This adaptability is further reinforced by the integration of Electronic Health Record (EHR) data and task/stimulation parameters, all of which provide ongoing intelligence for accelerated rehabilitation and long-term monitoring.

These operations are not merely mental steps; they require specialized computer modules to collect multimodal data, isolate speech signals, process utterances, validate against stored parameters, assign and store structured outputs, and integrate profiles with database systems. The proposed invention thus provides a concrete technological solution to the longstanding problem of ensuring accurate, repeatable, and adaptive speech pathology assessment. Therefore, the proposed system and method of the invention do not merely automate a known manual process. They ensure that speech data is collected, processed, profiled, and scored against predetermined task targets, with profiles used both as diagnostic outputs and as feedback to the_processing step. By coordinating analysis and profiling with the processing unit in real time, the system evaluates target parameters and conditions for subsequent steps, thereby ensuring alignment and integrity of diagnostic outcomes. Data is exchanged and stored across modules in local or remote databases, enabling scalability. Technical means of implementation and coordinated operations are thus provided.

Further features and particularities are defined in further sub-claims, with incorporation therein of (a) explicit references to computing means and (b) technical result in real devices, whereas further properties of the present invention will emerge from the description hereafter of some embodiments thereof.

### Brief description of the drawings

Fig. 1 shows a general structural and operational/functional diagram and flows of a main embodiment of the-Method according to the present invention as illustrated with its successive steps esp. for an Automated, Adaptive and Intelligent Profiling of Speech Responses in Mapping, Diagnosis, Treatment and Rehabilitation;
Fig. 2 shows specific interconnected functional blocks in the general structural and operational/functional diagram and flows of said main embodiment according to Fig. 1;

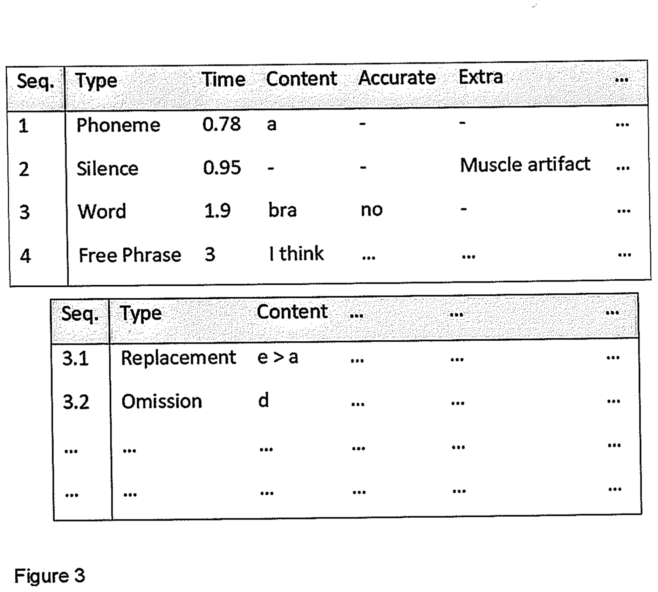

Fig. 3 shows a comparative table of experimental results of the-Method according to the present invention esp. of said main embodiment according to Fig. 1;

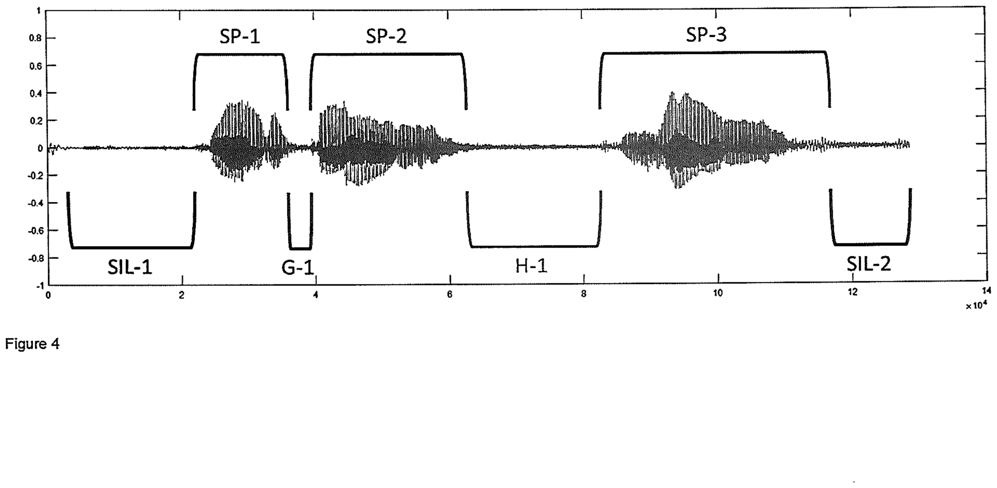

Fig. 4 shows a graph of experimental results of the-method according to the present invention esp. of said main embodiment according to Fig. 1.

### Description

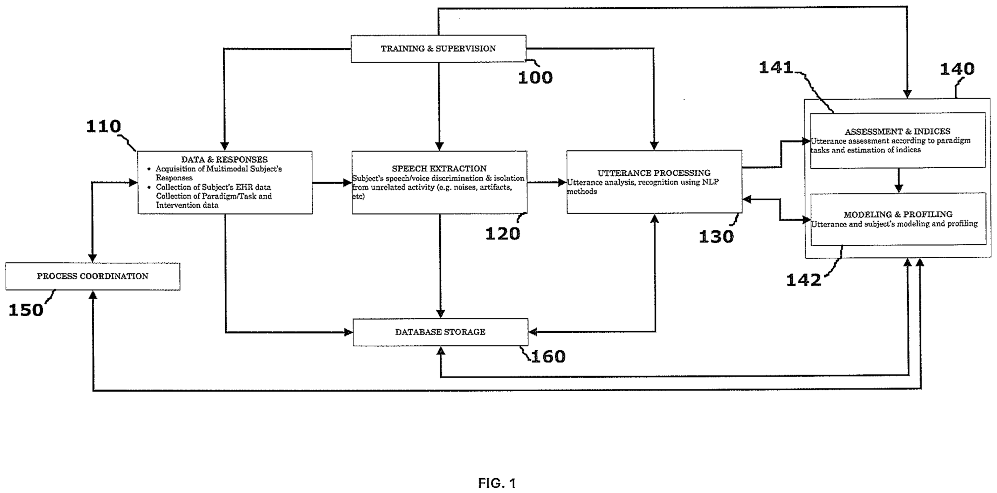

A general structural and operational/functional diagram of a method for an Automated, Adaptive and Intelligent Profiling of Speech Responses in Mapping, Diagnosis, Treatment and Rehabilitation comprising successive steps is shown in Fig. 1 and flows as follows, wherein the method comprises the following steps.

Step 1 refers to so-called data & responses 110 wherein subject multimodal raw data related to task and/or stimulation/testing regarding speech-related responses, e.g. speech/voice recordings, video recordings of facial/motional/positional/gestural state, vibratory state, neuro-muscular activity, together with Electronic Health Record EHR data, and task/paradigm/stimulation data are collected and used in subsequent steps. This data & responses step 110 notably includes the Acquisition of Multimodal Subject's Responses, the Collection of Subject's HER data and the Collection of Paradigm Task and Intervention data.

Step 2 refers to speech extraction 120, wherein the subject's speech/voice signals are detected, identified, discriminated and isolated from any unrelated activity e.g. noises, artifacts, and fed forward to the next step. This speech extraction step 120 thus notably includes subject's speech/voice discrimination & isolation from said unrelated activity.

Step 3 refers to utterance processing 130, wherein the extracted speech signal is processed and analyzed using NLP methods and techniques and detailed utterance structure is obtained, in terms of transcribed content, timings, phonetics, identification of singular events, e.g. hesitations, elongations. This step 130 notably includes Utterance analysis, recognition using NLP methods.

In Step 4 two functions are performed in parallel:
- a first assessment and indices function 141, wherein the detailed utterance description, content, structure, and events are evaluated and quantified against the predetermined task/paradigm targets/objectives, offering a characterization and scoring for the specific subject's performance. This function 141 notably includes Utterance assessment according to paradigm tasks and estimation of indices; and
- a second modeling & profiling function 142, wherein the same detailed utterance description, content, structure, events are fed to a modeling, classification, and profiling system that generates profiles of utterance specific, subject specific and population specific performance as well as profiles of speech characteristics, in conjunction to the various health, demographics, task/paradigm and stimulation parameters. This profiling is used both as an autonomous output and as feedback to the utterance processing step 130 aiming to improve accuracy and speed. This modeling & profiling function 142 notably includes Utterance and subject's modeling and profiling.

Step 5 refers to process coordination 150, wherein the results of the analysis and profiling are fed back to a processing unit 150 which appropriately evaluates in real-time the optimum parameters and conditions for the following steps of the subject's testing in order to maximize the informational content of the profiles at the individual subject and/or at population levels. For example, the processing unit 150 is able to decide on the content characteristics, presentation levels, timings regarding the sensory templates presented to the subject such as pictures, sounds, as well as parameters regarding possible external interventions to the subject's state e.g. said TMS, DCS or a type of stimulation/disturbance such as intensities, patterns, timings, spatial/volumetric distributions.

Fig. 1 also shows the corresponding system that employs extensive Machine Learning ML techniques at almost all steps. It is fully trainable and supervisable at all steps with the training & supervision module 100, it makes extensive use of decision making and can thus accumulate knowledge and constitute an intelligent system. Additionally, at all steps, data, inputs and outputs are exchanged with a complete local/remote/mixed database storage system through the database storage module 160.

### The ML model

The ML model is configured as a classifier or regression/prediction model which is trained with multimedia and multimodal input data. Its advantage over traditional static methods is the ability to identify complex, non-linear patterns/motifs in multidimensional data.

Its main feature is the unification, processing and inference on the basis of heterogeneous data sources such as:
a. Demographic data (age, gender, education),
b. Clinical data (EHR) (e.g. diagnoses, treatments,),
c. Parameters/Elements of the patterns of processes/naming tasks (e.g. characteristics of images, sounds, to be named),
d. Parameters/Elements of brain stimulation,
e. Results and errors from previous naming processes or other tests, often in the form of sequential data, feature vectors.

The model continuously accumulates newly collected data such as the above.

The output of the model consists of statistical models of speech/speaker profiles under various conditions, such as demographic data, EHR, stimulation parameters and patterns of naming procedures (e.g. prediction of possible pathology, assessment of severity of a condition, categorization of types/profiles of speech/speaker disorders). It also extracts/produces/creates adaptive settings of the parameters of the naming and brain stimulation procedures [parameters of patterns/paradigms of the naming tasks/procedures, parameters of flow/execution of the tasks/procedures, e.g. presentation times,].

### Structure and Architecture of ML

The model is structured as a multi-input regression/prediction and/or classification computation system (multi-input classifier/regressor). It asynchronously processes heterogeneous sources such as demographic data, EHR, time series (e.g. reaction times, error sequences,. from naming tests), stimulation parameters,.

The data are structured as tables (tabular data). The ML model at its core includes any of (or combination thereof of) the following techniques/algorithms and their respective architectures:
- Gradient Boosting Machines (e.g. XGBoost,).
- Random Forests
- Support Vector Machines (SVMs).

Especially at architecture level, this is defined by parameterizations such as:
- Random Forests: number of trees (n_estimators), maximum depth of tree (max_depth), criterion of split (e.g. Gini/Entropy), etc. The structure is a forest of parallel, independent trees.
- Gradient Boosting Machines: learning rate (learning_rate), maximum depth of trees, loss function (objective),. The structure is a (boosted) sequence of dependent trees, where each subsequent tree corrects the residuals of the previous one.
- Support Vector Machines (SVMs): Type of kernel functions (kernel) (e.g. linear, RBF, polynomial), separation criteria,.

The model is initially fed with a set of pre-existing/early data (such as those described above), and the optimal hyper-parameter values as well as the values of the respective coefficients/computation parameters are initialized based on these pre-existing/early data.

During the use of the system, the newly collected data are accumulated in the Database of the system, their processing and the derivation of results are performed by the ML model in question. The system provides the possibility for both the data and the extracted results to be recycled cumulatively together with the previous data for re-adaptation and optimization of the ML model.

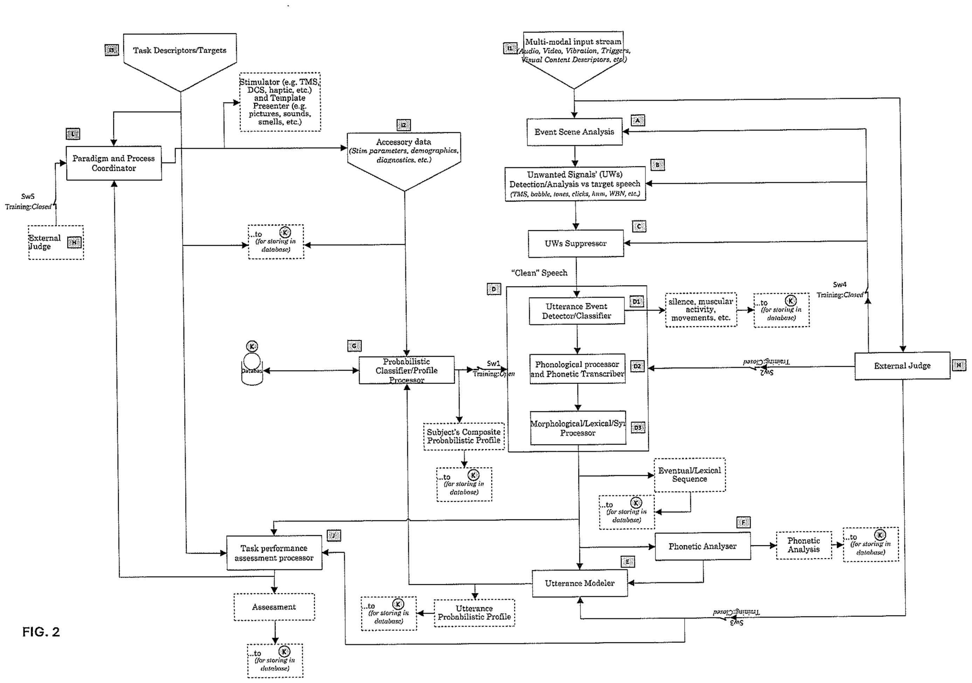

Fig. 2 shows the abovementioned system for an Automated, Adaptive and Intelligent Profiling of Speech Responses in Mapping, Diagnosis, Treatment and Rehabilitation, wherein the technology involved is implemented by means of a plurality of functional modules represented by blocks which are selectively interconnected, comprising the following:
an Intelligent Multimodal scene analysis is achieved by module A. Unwanted Signals' UWs, Detection/Analysis versus target speech (TMS, babble, tones, clicks, hum, WBN) is achieved by module B. UWs suppression is achieved by module C. Utterance Event Detection/Classification is achieved by module D1.

Multimodally assisted speech transcription and NLP processing (morphological analysis, concurrent lexical, syntactic, semantic and pragmatic analysis and annotation of the whole subject's utterance), and subsequent probabilistic prioritization of results that correspond to the speaker's intentioned response to the task through module D3 is achieved by Database storage and recall system through module K is achieved by Utterance and speaker's Probabilistic Classification/Profile Processor, for an intelligent determination of ecological qualitative and quantitative measures of clinical/pathognomonic interest, especially with the inclusion of additional input of conditional probabilistic expectancies such as subject's demographics, session place/time/duration details, naming tasks. Parameters/components/templates, intervention's parameters/components/templates such as brain stimulation process and parameters are achieved through module G. Adaptive Paradigm and Process Coordination, which adaptively determines the optimal naming tasks/paradigm's conduction parameters, as well as interventions parameters such as Brain stimulation of external experts like judge parameters, information rules/targets according to specific statistical or information rules/targets are achieved through module I.

The incorporation of accumulation of external expert's (judge) review and assessment of any system's obtained results, speaker's/utterance's profiles, determined parameters of naming tasks' execution - namely stimuli, templates, timings such as Brain stimulation parameters, and consequent fine-tuning of all the algorithmic procedures, targets assessments/corrections- are achieved through module H. The input data consist notably of Multi-modal input stream notably Audio, Video, Vibration, Triggers, Visual Content Descriptors, achieved through module 11 and Accessory data (Stim parameters, demographics, are achieved through module I2 and External Judger's Review/Assessment data are achieved through module H.

This general scheme of the system is thus realized by specific interconnected functional blocks of the Structural and Operational/Functional Flow as shown in Fig. 2 and recapitulated in Table 1 below.

**Table 1**

| **Block** | **Name** | **Description** |
|---|---|---|
| 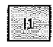 | Multi-modal input stream *(Audio, Video, Vibration, Triggers, Visual Content Descriptors)* | The stream of module I1 constitutes all raw signal data collected as subject responses, reactions, attitudes, performance, as well as any task specific data/metadata, regarding the auditory, visual, olfactory, gestural, tactile: stimuli, e.g. visualized object name, color/acoustic spectra, timings. However, the stream of module I1 may also contain other undesirable signals that may intrude during the signal/data acquisition process, such as noises, disturbances. |
| 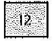 | Accessory data *(Stim parameters, demographics, diagnostics)* | Module I2 constitutes all additional data besides module I1, namely external brain stimulation parameters, e.g. rate, amplitudes, spatial/temporal distributions, subject's demographics, HER. |
| 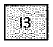 | Task Descriptors/Targets | The block I3 predefines the required quantitative and qualitative indices of interest, e.g. timings, intensities, as well as the targets, e.g. accurate repetition or naming of the specific experimental paradigm. |
| 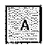 | Event Scene Analysis | The block A performs analysis of the scene, namely identification of the events that are captured by the various sensors, e.g. cameras, microphones, accelerometers; and whose signals are contained in the stream 11. Thus, block A performs an analysis of events regarding the subject's responses and/or any additional external event that may take place in the room and is contained in main stream I1. |
| | | The aim is to identify and classify any eventful source of information that is contained in stream I1, together with its spectro-temporal characteristics, in order to facilitate discretization of events. |
| | | It produces a table of named/classified or unnamed/unclassified events, together with their timings, and spectro-temporal signal characteristics or raw recording. The listed events can include any external source like sounds, e.g. TMS artifacts, any type of babbles, tones, clicks, beeps, sweeps, hum, WBN or other sounds, speech; lighting/visual events, e.g. flashes, dims, fluctuations, colorations; vibration/motion events, e.g. pushes, deliberate/undeliberate motions/vibrations, muscular artifacts; and in general any type of source that may intrude the recorded stream of raw signal data. The block A prepares data for the next block B. |
| | | The block A may employ ML (Gaussian mixture models GMM, hidden Markov models HMM and support vector machines SVM and DL, such as the convolutional neural network CNN and recurrent neural network RNN as well as the Bayesian generative model of sound) methods for Sound Event Detection SED, based on any JTFA data and/or psychoacoustical models/criteria for feature |
| | | generation/extraction from the input stream and subsequent scene's object classification [62]-[64], [108]-[113] |
| 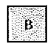 | Unwanted Signals' UWs Detection/Analysis vs target speech *(TMS, babble, tones, clicks, hum, WBN)* | Block B performs the discrimination between target-of-interest TOI responses/reactions from the subject and any other eventful signals which are not of interest and constitute Unwanted signals UWs that need to be eliminated so that the said TOI can be isolated and feasible to further analyze. |
| | | Mostly, based on block's A output, block B is able to discriminate said TOI from other disturbing or competing signals in the raw data, using intelligent decision criteria, as well as events' characteristics, e.g. spectro-temporal and any possible previously acquired data/ characteristics of said TOI, e.g. individual subject's speech samples/characteristics, [60], [61], [114], [115] |
| 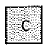 | UWs Suppressor | Block C suppresses/eliminates the said UWs that were determined in block B, employing a variety of signal processing algorithms individually for each and any kind of the said UWs. |
| | | The output of block C is a "clean" version of the said TOI, that contains an isolated and as purified as possible utterance speech from the subject for the subsequent analyses. |
| | | The block C also performs overall conditioning of the "clean" speech signal, e.g. filtering, amplitude conditioning. |
| | | Block C may employ algorithms for suppression of transient or continuous (stationary/nonstationary) signals [50], [51], [116]-[120]. |
| 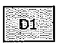 | Utterance Event Detector/Classifier | Block D1 detects, identifies, classifies and segregates the parts of the utterance in terms of phonemic/phonological units, and performs a logical classification of content as events (e.g., pure speech, silence, hesitant speech; and outputs a table with timings and nature of the utterance, as in Figure 3. Block D1 utilizes Voice Activity Detection VAD algorithms as well as Deep Neural Network's DNN or Statistical/Probabilistic Classifiers [121], [122]. Additionally, any prior statistical knowledge of the subject's speech profile is also incorporated as feedback from block F. |
| 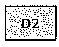 | Phonological processor and Phonetic Transcriber | Block D2 transcribes the speech units (silence is excluded) identified in block D1 into phonetic symbols and strings, using various methods [68]-[72], [84], following any probabilistic hierarchy that has been yielded by block D1. |
| 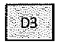 | Morphological/Lexical/S yntactic/Semantic/Prag matic Processor | Block D3 employing methods of NLP, serves as the main processing unit which performs morphological analysis, concurrent lexical, syntactic, semantic, and pragmatic analysis and annotation of the whole subject's utterance. It recognizes discrete behavioral sequences of speech and language units such as phonemes, words, syllables, phrases (complete or incomplete) various phonetic events e.g. phonemes/word parts elongations, or other segmental/suprasegmental speech units/events and recognizes through syntactic/pragmatic analysis the target word/phrase. |
| | | Following the probabilistic nature of the results of blocks D1 and D2, block D3 respectively produces probabilistic hierarchies of results that contain the identifications and marks of the parts of the utterance that correspond to the speaker's intentioned response to the task. |
| | | In a similar probabilistic hierarchy, block D3 outputs a complete table of fully time marked, transcribed, lexically, syntactically, semantically and pragmatically annotated written content of the subject's utterance [66], [68]-[73], [80]-[82], [84], [123]-[133], [5]. |
| | | It is emphasized that block D3 is able to operate well not only on the audio of the speech signal, but also in a combined stream of multimodal signals, such as audio-visual, e.g. audio together with video recording of gestural, facial, motional states, vibrational, neuro-muscular. |
| 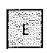 | Utterance Modeler | Block E builds sequenced, layered, object oriented ontological models of the utterance, as shown in Figure 4 and described by tables as in Figure 3, for use in block G. [8], [94]-[99], [134], [135]. The models also inherit a probabilistic hierarchical nature from the preceding analysis in the previous blocks, especially A, ..., D. |
| 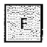 | Phonetic Analyzer | Block F performs phonetic analysis of each part of the utterance, by identifying and quantifying any phonetic parameters of interest. It is able |
| | | to include both directly estimated measures/attributes/indices of speech/voice acoustics as well as measures derived by employing perceptual/psychophysical criteria/models/techniques. [85]-[87], [136]-[138]. Block F also follows probabilistic hierarchy from the previous blocks, especially A, ..., E. |
| Ⓚ | Database | A local or remote or mixed database system for storing and exchanging data, inputs and outputs with all blocks. |
| 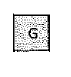 | Probabilistic Classifier/Profile Processor | Block G uses inputs from blocks K, E, F, I2 and I3 and employs statistical and/or deep learning techniques for the classification of the utterance given the characteristics of the subject's EHR, task parameters, etc., into discrete categories/profiles. |
| | | This serves both for depicting ecological qualitative and quantitative measures of clinical/pathognomonic interest, e.g. prevalence of misses in specific types of aphasias, in patients' populations, as well as any additional input of conditional probabilistic expectancies for improving the analysis in block D. |
| | | As mentioned, the registered and exchanged information with the database block K -in the form of records and fields or as object-oriented structures- includes any information collected from blocks I2, I3, and generated by blocks K, E, F such as subject's demographics, session place/time/duration details, paradigm/task parameters/components/ templates, interventional parameters/com ponents/templates. |
| | | The profiles are created by any possible way of combining and aggregating any of the above information types. |
| | | Block G may employ a variety of methods and techniques such as supervised/semi-supervised learning with deep structures, random forests, said SVM, etc. [88]-[93], [134], [135], [139]-[141] |
| | | As profiles are continuously updated with the accumulation of new data and analysis results from block D, E, F -for example in each presentation during a repetitive naming task-, and incorporating a decision-making step such as maximum likelihood selection or similar, block's G output is fed back to block D for further improving their |
| | | statistical performance, in terms of prior statistical knowledge of the subject's speech profiles. |
| 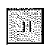 | External Judge | The object may be either a human or an intelligent machine-based judge that supervises and imposes correct values/elements as inputs or outputs of all blocks, and realizes the supervised/semi-supervised character of the respective operations. In conjunction with switches SW1, SW2, SW3, SW4, the system may be in independent running, - i.e. without judge interference or training mode, i.e. implied judge interference. |
| 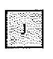 | Task performance assessment processor | Block I takes inputs from blocks D3, F and task targets and quantifies the various parameters of interest POI as well as the degree of conformance -in terms of accuracy or repeatability, namely types and numbers of divergences/errors from the specified task targets- by employing rules for decision making and indices of textual distances [45], [46], [100]-[104], [142], [143] |
| 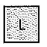 | Paradigm and Process Coordinator | Block L is able to adaptively coordinate the task's/paradigm's presentation and/or intervention parameters, according to specific statistical or informational rules/targets. For example, inaccurate namings or repetitions from the subject in certain templates, perceptual stimuli or interventional parameters, e.g. TMS intensity may require specific selection of their state values in order to obtain more detailed profiling of the subject's responses. |
| | | In the case of interventional paradigms, such as those using external stimulation like TMS, DC or any other kind of intervention to the subject, block L will be able to suggest the values of the stimulation parameters and the parameters or content of the presented templates to subject, such as sounds, pictures, or even directly control the appropriate software/hardware systems which perform the stimulation and template presentation. Similarly, in other testing or rehabilitation procedures which do not employ stimulation of the subject, such as in speech and language therapy, block L will still coordinate any template presentation parameters, such as rate of presentation (e.g. number of words per minute), its content e.g. selection of the specific word type and phonemic, semantic, intonation, or prosodic content; the intensity levels, and any other parameter for the process. In cases where the process is performed using automated systems, block L will also directly control their parameters, interface. |

Table 2 below summarizes the various functionalities and advantages offered by the claimed design, together with reference to the named blocks of Fig. 2.

**Table 2**

| **No** | **Functionality** | **Named block (****Figure 2****)** |
|---|---|---|
| 1 | Coverage of any type of speech-based response paradigm/task | 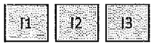 |
| 2 | Analysis and Decision Intelligence | all |
| 3 | Detection, identification and qualitative and quantitative characterization of acoustic events, verbal content, and extraction of landmark estimates and signal characteristics | 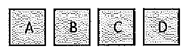 |
| 4 | Real-time analysis and scoring | all |
| 5 | Acquisition and integration of multimodal (e.g., acoustic, visual, gestural, vibratory, electrophysiological, neuromuscular, etc.) data and collection of any type of medical information or manipulative parameters (e.g., stimulation, type/presentation of examples namely visual, auditory) | 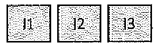 |
| 6 | Robustness to violations and impurities of Target of Interest signals | 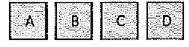 |
| 7 | Thorough NLP analysis | 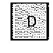 |
| 8 | Exploitation of State-of-the-art computational technologies into an evolving infrastructure | all |
| 9 | Patient individualization and online adaptation to running assessment of responses/profiles | 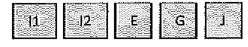 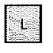 |
| 10 | Utterance and speaker profiling | 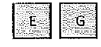 |
| 11 | Versatility and robustness to various types of phonological differentiations (e.g., types of aphasia, stimulation types) | 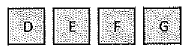 |
| 12 | Incorporation of accumulated prior knowledge of utterance/speaker profiles as feedback into the speech analysis | 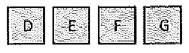 |
| 13 | Integration to Brain Studies (e.g., stimulation), cognitive testing or any SLP schema | all |
| 14 | Intelligent speech's model Construction and classification, under any type of task, paradigm (e.g., experimental or manipulative condition) or speech conditions (e.g., aphasic, dysarthric) | 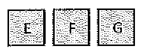 |
| 15 | Independent, Trainable or Mixed operation | all |
| 16 | Supervision by Expert Judge (Human or Machine-based) | 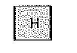 |

### Example

A typical example of application consists of the Analysis and Profiling of Speech Responses in a Picture Naming Task under said TMS. In this specific variant of the picture naming task, the main aim is to assess the subject's ability to name pictures under TMS stimulation, serving purposes of brain mapping of speech production structures.

The task involves two phases Φ₁, Φ₂. In the first phase Φ₁ (Phase 1), which does not include any TMS stimulation, a form of the subject's "baseline" performance characteristics is acquired, by presenting the subject with pictures and the latter is asked to name them, and the responses are recorded and analyzed, in order to provide the tester or operator with reference performance values and information on appropriate parameterization of the process. The second phase Φ₂ (Phase 2) constitutes the main brain mapping paradigm using TMS, which is performed after the specification of the paradigm's parameters based on the results of the first phase Φ₁,

In more detail, the first phase Φ₁ (Phase 1) serves two main goals:
a) to identify all pictures associated with abnormal subject's naming responses -that is, incorrectly named or named with hesitation or unnamed- so that they are removed from the presentation material of the subsequent actual mapping, and
b) to adjust the interpicture interval (IPI) and the picture presentation time (PPT), so that they fit with the subject's performance ability. Notably, both goals (i.e. identification of abnormal responses and adjustment of the optimal, personalized IPI & PPT) are currently attained manually, which is laborious, operator-dependent and prone to error. The incorporation of the hereby proposed method, after the automated and intelligent processes of "cleaning" recorded responses from unwanted intruding signals, and speech analysis and recognition (Figure 1, Step 1, Step 2, Step 3) provides the desired information for said goal a. (Fig. 1, Step 4.1, and results from Figure's 2 block J-"Task performance assessment processor"), and for said goal b. (Fig. 1, Step 5, and results from Fig.'s 2 block L-"Paradigm and Process Coordinator"). As a result we accomplish the rapid and objective identification of abnormal responses and the precise, customized algorithmic adjustment of IPI & PPT. Concretely, response characteristics, such as the latency of uttering the name for the picture (time difference of the uttering time from the picture presentation time), pace and tone of speech, are registered at each trial. The collection of the values for each such characteristic forms the distribution of the characteristic at normal response. This will serve as the template for determining normal and abnormal responses, the latter induced by nr-TMS in the subsequent Phase 2. More specific classifications of abnormal response are also defined as explained below, in the description of Phase 2. Note that the normal uttering/speech characteristic distributions are subject-specific and adapted to the designed IPI and PPT.

In Phase 2, the patient is presented with the remaining presentation material (not discarded after Phase 1) while nr-TMS stimulation is performed over a wide cortical region in the perisylvian area, with particularly dense spatial sampling perilesionally, and typically in synchrony with the appearance of pictures (picture-to-rTMS trigger interval (PTI)= 0ms). Typically, 40-80 sites in each hemisphere are stimulated at three nonconsecutive times each, resulting in a total of 120-240 stimulations per hemisphere. It should be noted that in general stimulation of both hemispheres is recommended (i.e. both the lesion-harboring hemisphere as well as the contralateral one) so as to identify lesion-induced plasticity phenomena. The response to each stimulus is classified as normal or abnormal and in the latter case it is further classified as no response (also known as speech arrest or aphasic anomia), performance error (including dysarthria or speech apraxia), hesitation, circumlocution, paraphasias (semantic or phonological) and neologisms. It should be stressed that the final stimulation paradigm employed in Phase 2 is adaptive, meaning that the full parametrization of the procedure (including stimulus intensity and frequency, number of stimuli, IPI, PPT and PTI). Notably, the full parametrization of the stimulation paradigm is currently performed manually, which is laborious, operator-dependent and prone to error. The incorporation of the hereby proposed method in the previous Phase 1, based on the speed and accuracy of the automated extraction of results, facilitates the rapid and objective identification of abnormal responses and thereby permit the precise, customized parametrization of the full set of stimulation parameters. Concretely, through a training period of Phase 1 and 2, threshold values defining the tails of the distribution of each normal uttering/speech characteristic -at very low significance level- are set. These threshold values determine the deviation from normal of each speech characteristic and classify in terms of each characteristic the response as normal or abnormal.

Typically in current practice, the video-recorded responses of the subject from Phase 2 are analyzed by neurolinguists -or other relevant personnel- and each response is again classified as normal or abnormal and in the latter case it is further classified as no response (also known as speech arrest or aphasic anomia), performance error -including dysarthria or speech apraxia-, hesitation, circumlocution, paraphasias -semantic or phonological- and neologisms. Every brain stimulation site where an error is identified at least two nonconsecutive times is regarded as nr TMS-positive and indicates functional, language-related brain tissue. Notably the identification of abnormal responses is currently performed manually, which is laborious, operator-dependent and prone to error. The incorporation of the hereby proposed method will result in the real-time, rapid and objective identification of abnormal responses as represented in module 141 of Fig. 1, Step 4.1, and resulting from module J for "Task performance assessment processor" of Fig.'s 2, and the creation of detailed speaker's utterance profile as represented in module 142 of Fig. 1, Step 4.2, and resulting from Fig.'s 2 module G for "Probabilistic Classifier/Profile Processor" and subsequent construction of the subject's language network brain map.

Additionally, the outcomes of the present automated adaptive method -especially the decision as normal or abnormal of each uttering/speech characteristic- at each of the three repetitions, will allow the realization of fusion schemes to decide whether the overall response will be designated as normal or abnormal, and even give a score of abnormality based on the decision on each of the specific abnormality types: no response (also known as speech arrest or aphasic anomia), performance error -including dysarthria or speech apraxia-, hesitation, circumlocution, paraphasias (semantic or phonological) and neologisms.

### Key expressions

acoustic event, acoustic scene analysis, acoustic scene segregation, adaptive testing, apraxia of speech, audio-visual speech recognition, auditory processing, automated speech recognition, background noise, brain damage, brain injuries, brain network, brain stimulation, brain stimulation research, clinical natural language processing, computational linguistics, deep learning, deep neural networks, direct brain stimulation, individualization, language disorders, language production, language therapy, lexical analysis, machine learning, motor speech disorder, motor speech disorders, multi-modal input, natural language processing, neural networks, neural probabilistic language model, non-invasive brain stimulation, ontological analysis, speaker-dependent speech recognition, phonetic analysis, phonological processing, pragmatic analysis, process coordination, real-time transcription, semantic context, semantic processing, speaker profiling, speech articulation, speech audiometry, speech perception, speech production, speech quality perception, speech representations, speech sound disorders, speech-in-noise testing, syntactic analysis, timing of speech.

### REFERENCES

[1] J. S. Damico, N. Müller, and M. J. Ball, Eds., The Handbook of Language and Speech Disorders, 2nd edition. Hoboken, NJ: Wiley-Blackwell, 2021.
[2] I. Papathanasiou and P. Coppens, Aphasia and Related Neurogenic Communication Disorders, 2nd edition. Burlington, MA: Jones & Bartlett Learning, 2016.
[3] R. H. B. P. CCC/SP and M. R. M. P. C.-S. BC-NCD, Introduction to Neurogenic Communication Disorders, 8th edition. St. Louis, Missouri: Mosby, 2014.
[4] I. Reinvang, Aphasia and Brain Organization. Boston, MA: Springer US, 1985. doi: 10.1007/978-1-4757-9214-0.
[5] C. Themistocleous, K. Webster, A. Afthinos, and K. Tsapkini, "Part of Speech Production in Patients With Primary Progressive Aphasia: An Analysis Based on Natural Language Processing," Am. J. Speech Lang. Pathol., vol. 30, no. 1 Suppl, pp. 466-480, Feb. 2021, doi: 10.1044/2020_AJSLP-19-00114.
[6] K. Grechuta et al., "Multisensory cueing facilitates naming in aphasia," J. NeuroEngineering Rehabil., vol. 17, no. 1, p. 122, Dec. 2020, doi: 10.1186/s12984-020-00751-w.
[7] G. Hickok and D. Poeppel, "The cortical organization of speech processing," Nat. Rev. Neurosci., vol. 8, no. 5, Art. no. 5, May 2007, doi: 10.1038/nrn2113.
[8] S. Vázquez González and M. Somodevilla Garcia, "A computational model for speech disorders using problematic phonemes with ontological reasoning," J. Intell. Fuzzy Syst., vol. 39, no. 2, pp. 2305-2315, Jan. 2020, doi: 10.32331/JIFS-179892
[9] I. Rujas, S. Mariscal, E. Murillo, and M. Lázaro, "Sentence Repetition Tasks to Detect and Prevent Language Difficulties: A Scoping Review," Children, vol. 8, no. 7, p. 578, Jul. 2021, doi: 10.3390/children8070578.
[10] G. H. Poll, C. A. Miller, and J. G. van Hell, "Sentence Repetition Accuracy in Adults With Developmental Language Impairment: Interactions of Participant Capacities and Sentence Structures," J. Speech Lang. Hear. Res. JSLHR, vol. 59, no. 2, pp. 302-316, Apr. 2016, doi: 10.1044/2015_JSLHR-L-15-0020.
[11] E. Theodorou, M. Kambanaros, and K. K. Grohmann, "Sentence Repetition as a Tool for Screening Morphosyntactic Abilities of Bilectal Children with SLI," Front. Psychol., vol. 8, 2017, Accessed: Jul. 30, 2023. [Online]. Available: https://www.frontiersin.org/articles/10.3389/fpsyg.2017.02104
[12] S. Reeves et al., "Narrative video scene description task discriminates between levels of cognitive impairment in Alzheimer's Disease," Neuropsychology, vol. 34, no. 4, pp. 437-446, May 2020, doi: 10.1037/neu0000621.
[13] K. J. Tomlin, WALC. 8, Word finding: workbook of activities for language and cognition. East Moline, IL: LinguiSystems, 2007.
[14] A. Abad et al., "Automatic word naming recognition for an on-line aphasia treatment system," Comput. Speech Lang., vol. 27, no. 6, pp. 1235-1248, Sep. 2013, doi: 10.1016/j.csl.2012.10.003.
[15] A. E. Tervo et al., "Closed-loop optimization of transcranial magnetic stimulation with electroencephalography feedback," Brain Stimulat., vol. 15, no. 2, pp. 523-531, Mar. 2022, doi: 10.1016/j.brs.2022.01.016.
[16] P. Lioumis et al., "Study Design for Navigated Repetitive Transcranial Magnetic Stimulation for Speech Cortical Mapping," J. Vis. Exp. JoVE, no. 193, Mar. 2023, doi: 10.3791/64492.
[17] E. Maas et al., "Principles of Motor Learning in Treatment of Motor Speech Disorders," Am. J. Speech Lang. Pathol., vol. 17, no. 3, pp. 277-298, Aug. 2008, doi: 10.1044/1058-0360(2008/025).
[18] R. Landin-Romero et al., "Brain changes underlying progression of speech motor programming impairment," Brain Commun., vol. 3, no. 3, p. fcab205, Sep. 2021, doi: 10.1093/braincomms/fcab205.
[19] A. Morgan, F. Liégeois, and F. Vargha-Khadem, "Motor speech profile in relation to site of brain pathology: a developmental perspective," in Speech Motor Control: New developments in basic and applied research, B. Maassen and P. van Lieshout, Eds., Oxford University Press, 2010, p. 0. doi: 10.1093/acprof.oso/97801882356797.003.0006.
[20] A. Morgan, F. Liégeois, and F. Vargha-Khadem, "Motor speech outcome as a function of the site of brain pathology: A developmental perspective," in Speech motor control: New developments in basic and applied research, Oxford University Press, 2010, p. pp.95-115.
[21] L. Brabenec, J. Mekyska, Z. Galá 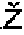 , P. Klobu 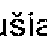 iaková, M. Ko 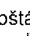 tálová, and I. Rektorova, "Effects of non-invasive brain stimulation on motor speech disorder in Parkinson's disease," *Clin. Neurophysiol.,* vol. 129, p. e9, Apr. 2018, doi: 10.1016/j.clinph.2018.01.030.
[22] M. Landry, J. da Silva Castanheira, and K. Jerbi, "Differential and Overlapping Effects between Exogenous and Endogenous Attention Shape Perceptual Facilitation during Visual Processing," J. Cogn. Neurosci., vol. 35, no. 8, pp. 1279-1300, Aug. 2023, doi: 10.1162/jocn_a_02015.
[23] Y. Zhang, J. Zou, and N. Ding, "Complex Mapping between Neural Response Frequency and Linguistic Units in Natural Speech," J. Cogn. Neurosci., vol. 35, no. 8, pp. 1361-1368, Aug. 2023, doi: 10.1162/jocn_a_02013.
[24] S. Pinet and N. Nozari, "Different Electrophysiological Signatures of Similarity-induced and Stroop-like Interference in Language Production," J. Cogn. Neurosci., vol. 35, no. 8, pp. 1329-1349, Aug. 2023, doi: 10.1162/jocn_a_02014.
[25] T. O. Bergmann and G. Hartwigsen, "Inferring Causality from Noninvasive Brain Stimulation in Cognitive Neuroscience," J. Cogn. Neurosci., vol. 33, no. 2, pp. 195-225, Feb. 2021, doi: 10.1162/jocn_a_01591.
[26] L. Rodrigues de Almeida, P. A. Pope, and P. C. Hansen, "Task load modulates tDCS effects on brain network for phonological processing," Cogn. Process., vol. 21, no. 3, pp. 341-363, Aug. 2020, doi: 10.1007/s10339-020-00964-w.
[27] G. I. De Zubicaray and V. Piai, "Investigating the Spatial and Temporal Components of Speech Production," in The Oxford Handbook of Neurolinguistics, G. I. De Zubicaray and N. O. Schiller, Eds., Oxford University Press, 2019, pp. 471-497. doi: 10.1093/oxfordhb/9780190672027.013,19.
[28] V. Sreekumar, J. H. Wittig, T. C. Sheehan, and K. A. Zaghloul, "Principled Approaches to Direct Brain Stimulation for Cognitive Enhancement," Front. Neurosci., vol. 11, 2017, Accessed: Jul. 30, 2023. [Online]. Available: https://www.frontiersin.org/articles/10.3389/fnins.2017.00650
[29] W. Medeiros, T. Barros, and F. V. Caixeta, "Bibliometric mapping of non-invasive brain stimulation techniques (NIBS) for fluent speech production," Front. Hum. Neurosci., vol. 17, 2023, Accessed: Jul. 30, 2023. [Online]. Available: https://www.frontiersin.org/articles/10.3389/fnhum.2023.1164890
[30] A. Buchwald, H. Calhoun, S. Rimikis, M. Steinberg Lowe, R. Wellner, and D. Edwards, "Using tDCS to facilitate motor learning in speech production: The role of timing," Cortex J. Devoted Study Nerv. Syst. Behav., vol. 111, pp. 274-285, Feb. 2019, doi: 10.1016/j.cortex.2018.11.014.
[31] T. Murakami, Y. Ugawa, and U. Ziemann, "Utility of TMS to understand the neurobiology of speech," Front. Psychol., vol. 4, p. 446, Jul. 2013, doi: 10.3389/fpsyg.2013.00446.
[32] P. Lioumis et al., "A novel approach for documenting naming errors induced by navigated transcranial magnetic stimulation," J. Neurosci. Methods, vol. 204, no. 2, pp. 349-354, Mar. 2012, doi: 10.1016/j.jneumeth,2011.11.003.
[33] A. Tsitlakidis et al., "Navigated rTMS for Mapping the Language Network in Preoperative Settings: Current Status and Future Prospects," in Translational Neuroscience of Speech and Language Disorders, G. P. D. Argyropoulos, Ed., in Contemporary Clinical Neuroscience. Cham: Springer International Publishing, 2020, pp. 177-204. doi: 10.1007/978-3-030-35687-3_9.
[34] S. M. Krieg, Ed., Navigated Transcranial Magnetic Stimulation in Neurosurgery. Cham: Springer International Publishing, 2017. doi: 10.1007/978-3-319-54918-7.
[35] K. M. Grande, S. K. Z. Ihnen, and R. Arya, "Electrical Stimulation Mapping of Brain Function: A Comparison of Subdural Electrodes and Stereo-EEG," Front. Hum. Neurosci., vol. 14, 2020, Accessed: Sep. 06, 2023. [Online]. Available: https://www.frontiersin.org/articles/10.3389/fnhum.2020.611291
[36] S. Kovac, C. A. Scott, V. Maglajlija, R. Rodionov, A. W. McEvoy, and B. Diehl, "Extraoperative electrical cortical stimulation: characteristics of motor responses and correlation with precentral gyrus," J. Clin. Neurophysiol. Off. Publ. Am. Electroencephalogr. Soc., vol. 28, no. 6, pp. 618-624, Dec. 2011, doi: 10.1097/WNP.0b013e31823cc0f9.
[37] A. Suller Marti et al., "Extraoperative electrical stimulation mapping in epilepsy presurgical evaluation: a proposal and review of the literature," Clin. Neurol. Neurosurg., vol. 214, p. 107170, Mar. 2022, doi: 10.1016/j.clineuro.2022.107170.
[38] C. Isitan, Q. Yan, D. D. Spencer, and R. Alkawadri, "Brief history of electrical cortical stimulation: A journey in time from Volta to Penfield," Epilepsy Res., vol. 166, p. 106363, Oct. 2020, doi: 10.1016/j.eplepsyres.2020.106363.
[39] D. J. Caldwell, J. G. Ojemann, and R. P. N. Rao, "Direct Electrical Stimulation in Electrocorticographic Brain-Computer Interfaces: Enabling Technologies for Input to Cortex," Front. Neurosci., vol. 13, 2019, Accessed: Sep. 06, 2023. [Online]. Available: https://www.frontiersin.org/articles/10.3389/fnins.2019.00804
[40] G. Alarcon, L. Nawoor, and A. Valentin, "Electrical Cortical Stimulation: Mapping for Function and Seizures," Neurosurg. Clin. N. Am., vol. 31, no. 3, pp. 435-448, Jul. 2020, doi: 10.1016/j.nec.2020.03.013.
[41] K. James, The Strands of Speech and Language Therapy: Weaving Plan for Neurorehabilitation. Routledge, 2020.
[42] A. A. M. S. CCC-SLP and A. Anderson, Speech Therapy Aphasia Rehabilitation Workbook: Expressive and Written Language. CreateSpace Independent Publishing Platform, 2013.
[43] Pro-Ed, Speech and Language Rehabilitation - Fourth Edition. Pro-ED, 2001.
[44] R. Palmer and A. Pauranik, "Rehabilitation of Communication Disorders," in Clinical Pathways in Stroke Rehabilitation: Evidence-based Clinical Practice Recommendations, T. Platz, Ed., Cham (CH): Springer, 2021. Accessed: Jul. 30, 2023. [Online]. Available: http://www.ncbi.nlm.nih.gov/books/NBK585589/
[45] T. Frasca and M. Scheutz, "A Framework for Robot Self-Assessment of Expected Task Performance," IEEE Robot. Autom. Lett., vol. 7, no. 4, pp. 12523-12530, Oct. 2022, doi: 10.1109/LRA.2022.3219024.
[46] L. Kaldaras and K. C. Haudek, "Validation of automated scoring for learning progression-aligned Next Generation Science Standards performance assessments," Front. Educ., vol. 7, 2022, Accessed: Jul. 29, 2023. [Online]. Available: https://www.frontiersin.org/articles/10.3389/feduc.2022.968289
[47] C. A. Niziolek and S. Kiran, "Assessing speech correction abilities with acoustic analyses: Evidence of preserved online correction in persons with aphasia," Int. J. Speech Lang. Pathol., vol. 20, no. 6, pp. 659-668, Oct. 2018, doi: 10.1080/17549507.2018.1498920.
[48] N. E. Tomassi, H. R. Weerathunge, M. R. Cushman, J. W. Bohland, and C. E. Stepp, "Assessing Ecologically Valid Methods of Auditory Feedback Measurement in Individuals With Typical Speech," J. Speech Lang. Hear. Res., vol. 65, no. 1, pp. 121-135, Jan. 2022, doi: 10.1044/2021_JSLHR-21-00377.
[49] J. McKechnie, M. Shahin, B. Ahmed, P. McCabe, J. Arciuli, and K. J. Ballard, "An Automated Lexical Stress Classification Tool for Assessing Dysprosody in Childhood Apraxia of Speech," Brain Sci., vol. 11, no. 11, p. 1408, Oct. 2021, doi: 10.3390/brainsci11111408.
[50] A. Kaundanya, A. Anand, K. Raisinghani, and R. Sonkusare, "Analysis of Signal Noise Reduction Techniques," in Proceedings of Third International Conference on Communication, Computing and Electronics Systems, V. Bindhu, J. M. R. S. Tavares, and K.-L. Du, Eds., in Lecture Notes in Electrical Engineering. Singapore: Springer, 2022, pp. 425-437. doi: 10.1007/978-981-16-8862-1_28.
[51] R. Liang, Y. Xie, J. Cheng, G. Tang, and S. Sun, "Real-time speech enhancement algorithm for transient noise suppression," Multimed. Tools Appl., vol. 80, no. 3, pp. 3681-3702, Jan. 2021, doi: 10.1007/s11042-020-09849-8.
[52] V. Am, M. E, L. P, J. V, and M. Jp, "Accelerometer-based automatic voice onset detection in speech mapping with navigated repetitive transcranial magnetic stimulation," J. Neurosci. Methods, vol. 253, Sep. 2015, doi: 10.1016/j.jneumeth.2015.05.015.
[53] W. Wang, W. Wang, M. Sun, and C. Wang, "Acoustic Scene Analysis with Multi-Head Attention Networks," in Interspeech 2020, ISCA, Oct. 2020, pp. 1191-1195. doi: 10.21437/Interspeech.2020-1342,
[54] I. Martin-Morató, T. Heittola, A. Mesaros, and T. Virtanen, "Low-complexity acoustic scene classification for multi-device audio: analysis of DCASE 2021 Challenge systems." arXiv, Jul. 20, 2021. doi: 10.48550/arXiv.2105.13734.
[55] S. Teki, M. Chait, S. Kumar, S. Shamma, and T. D. Griffiths, "Segregation of complex acoustic scenes based on temporal coherence," eLife, vol. 2, p. e00699, Jul. 2013, doi: 10.7554/eLife.00699.
[56] S. Park, S. Mun, Y. Lee, D. K. Han, and H. Ko, "Analysis Acoustic Features for Acoustic Scene Classification and Score fusion of multi-classification systems applied to DCASE 2016 challenge".
[57] A. K. Mukhopadhyay, M. P. Naligala, D. L. Duggisetty, I. Chakrabarti, and M. Sharad, "Acoustic scene analysis using analog spiking neural network," Neuromorphic Comput. Eng., vol. 2, no. 4, p. 044003, Oct. 2022, doi: 10.1088/2634-4386/ac90e5.
[58] B. T. Szabó, S. L. Denham, and I. Winkler, "Computational Models of Auditory Scene Analysis: A Review," Front. Neurosci., vol. 10, 2016, Accessed: Jul. 30, 2023. [Online]. Available: https://www.frontiersin.org/articles/10.3389/fnins.2016.00524
[59] T. Virtanen, M. D. Plumbley, and D. Ellis, Eds., Computational Analysis of Sound Scenes and Events. Cham: Springer International Publishing, 2018. doi: 10.1007/978-3-319-63450-0.
[60] Y. Shao, S. Srinivasan, Z. Jin, and D. Wang, "A computational auditory scene analysis system for speech segregation and robust speech recognition," Comput. Speech Lang., vol. 24, no. 1, pp. 77-93, Jan. 2010, doi: 10.1016/j.csl.2008.03.004.
[61] G. J. Brown and D. Wang, "Separation of Speech by Computational Auditory Scene Analysis," in Speech Enhancement, J. Benesty, S. Makino, and J. Chen, Eds., in Signals and Communication Technology. Berlin, Heidelberg: Springer, 2005, pp. 371-402. doi: 10.1007/3-540-27489-8_16.
[62] K. Imoto, "Introduction to acoustic event and scene analysis," Acoust. Sci. Technol., vol. 39, no. 3, pp. 182-188, May 2018, doi: 10,1250/ast.39.182.
[63] K. Imoto and N. Ono, "Acoustic scene analysis from acoustic event sequence with intermittent missing event," in 2015 IEEE International Conference on Acoustics, Speech and Signal Processing (ICASSP), Apr. 2015, pp. 156-160. doi: 10.1109/ICASSP.2015.7177951.
[64] J. Ye, T. Kobayashi, N. Toyama, H. Tsuda, and M. Murakawa, "Acoustic Scene Classification Using Efficient Summary Statistics and Multiple Spectro-Temporal Descriptor Fusion," Appl. Sci., vol. 8, no. 8, Art. no. 8, Aug. 2018, doi: 10.3390/app8081363.
[65] G. K. Charitos, "Deep Learning Methods for Auditory Scene Analysis," M.Sc. Thesis, University of Athens, Athens, Greece, 2022.
[66] T. Afouras, J. S. Chung, A. Senior, O. Vinyals, and A. Zisserman, "Deep Audio-Visual Speech Recognition," IEEE Trans. Pattern Anal. Mach. Intell, vol. 44, no. 12, pp. 8717-8727, Dec. 2022, doi: 10.1109/TPAMI.2018.2889052.
[67] H. V. Sharma and M. Hasegawa-Johnson, "Acoustic model adaptation using in-domain background models for dysarthric speech recognition," Comput. Speech Lang., vol. 27, no. 6, pp. 1147-1162, Sep. 2013, doi: 10.1016/j.csl.2012.10.002.
[68] C. B. Fox, M. Israelsen-Augenstein, S. Jones, and S. L. Gillam, "An Evaluation of Expedited Transcription Methods for School-Age Children's Narrative Language: Automatic Speech Recognition and Real-Time Transcription," J. Speech Lang. Hear. Res. JSLHR, vol. 64, no. 9, pp. 3533-3548, Sep. 2021, doi: 10.104412021 JSLHR-21-00096.
[69] L. Harrington, "Incorporating automatic speech recognition methods into the transcription of police-suspect interviews: factors affecting automatic performance," Front. Commun., vol. 8, 2023, Accessed: Jul. 28, 2023. [Online]. Available: https://www.frontiersin.org/articles/10.3389/fcomm.2023.1165233
[70] A. Amberkar, P. Awasarmol, G. Deshmukh, and P. Dave, "Speech Recognition using Recurrent Neural Networks," in 2018 International Conference on Current Trends towards Converging Technologies (ICCTCT), Mar. 2018, pp. 1-4. doi: 10.1109/ICCTCT.2018.8551185.
[71] A. Graves, A. Mohamed, and G. Hinton, "Speech recognition with deep recurrent neural networks," in 2013 IEEE International Conference on Acoustics, Speech and Signal Processing, Vancouver, BC, Canada: IEEE, May 2013, pp. 6645-6649. doi: 10.1109/ICASSP.2013.6638947.
[72] A. Graves and N. Jaitly, "Towards End-to-End Speech Recognitionwith Recurrent Neural Networks," presented at the ICML, 2014.
[73] "HuBERT: Speech representations for recognition & generation." https://ai.facebook.com/blog/hubert-self-supervised-representation-learning-for-speech-recognition-generation-and-compression/ (accessed Jul. 28, 2023).
[74] L.Deng and Y.Liu, Eds, Deep Learning in Natural Language Processing, 1sted. 2018 edition New York NY Springer,2018
[75] C. D. Manning and H. Schütze, Foundations of Statistical Natural Language Processing, 1st edition. Cambridge, Mass: The MIT Press, 1999.
[76] Y. Goldberg, Neural Network Methods in Natural Language Processing. Morgan & Claypool Publishers, 2017.
[77] J. D. Kelleher, B. M. Namee, and A. D'Arcy, Fundamentals of Machine Learning for Predictive Data Analytics: Algorithms, Worked Examples, and Case Studies, 1st edition. Cambridge, Massachusetts: The MIT Press, 2015.
[78] I. Goodfellow, Y. Bengio, and A. Courville, Deep Learning. MIT Press, 2016.
[79] N. Grabar, C. Grouin, and Section Editors of the IMIA Yearbook Section on Clinical Natural Language Processing, "Year 2020 (with COVID): Observation of Scientific Literature on Clinical Natural Language Processing," Yearb. Med. Inform., vol. 30, no. 1, pp. 257-263, Aug. 2021, doi: 10,1055/s-0041-1726528.
[80] Aspects of Automatic Text Analysis, vol. 209. in Studies in Fuzziness and Soft Computing, vol. 209. Berlin, Heidelberg: Springer, 2006. doi: 10.1007/978-3-540-37522-7.
[81] A. Mehler, K.-U. Kühnberger, H. Lobin, H. Lüngen, A. Storrer, and A. Witt, Eds., Modeling, Learning, and Processing of Text Technological Data Structures, vol. 370. in Studies in Computational Intelligence, vol. 370. Berlin, Heidelberg: Springer, 2012. doi: 10.1007/978-3-642-22613-7.
[82] A. Storrer, "Lexical-Semantic Resources in Automated Discourse Analysis", Accessed: Jul. 29, 2023. [Online]. Available: https://www.academia.edu/69152800/Lexical_Semantic_Resources_in_Automated_Discourse_Analysis
[83] C. Leong and D. Whitenack, "Phone-ing it in: Towards Flexible, Multi-Modal Language Model Training using Phonetic Representations of Data".
[84] B. L 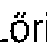 rincz, "Concurrent phonetic transcription, lexical stress assignment and syllabification with deep neural networks," *Procedia Comput. Sci.,* vol. 176, pp. 108-117, 2020, doi: 10.1016/j.procs.2020.08.012.
[85] N. Giovannone and R. M. Theodore, "Individual Differences in the Use of Acoustic-Phonetic Versus Lexical Cues for Speech Perception," Front. Commun., vol. 6, 2021, Accessed: Jul. 29, 2023. [Online]. Available: https://www.frontiersin.org/articles/10.3389/fcomm.2021.691225
[86] K. N. Stevens, Acoustic Phonetics. Cambridge, Mass.: The MIT Press, 2000.
[87] K. Johnson, Acoustic and Auditory Phonetics, 3rd edition. Malden, MA: Wiley-Blackwell, 2011.
[88] A. H. P. Alavijeh, "Speaker Profiling for Forensic Applications," M.Sc. Thesis, KU Leuven, Leuven, 2014.
[89] C. Müller, Ed., Speaker Classification II: Selected Projects, vol. 4441. in Lecture Notes in Computer Science, vol. 4441. Berlin, Heidelberg: Springer, 2007. doi: 10.1007/978-3-540-74122-0.
[90] C. Müller, Ed., Speaker Classification I: Fundamentals, Features, and Methods, vol. 4343. in Lecture Notes in Computer Science, vol. 4343. Berlin, Heidelberg: Springer, 2007. doi: 10.1007/978-3-540-74200-5.
[91] C. Fayet, A. Delhay, D. Lolive, and P.-F. Marteau, "Unsupervised Classification of Speaker Profiles as a Point Anomaly Detection Task," in Proceedings of the First International Workshop on Learning with Imbalanced Domains: Theory and Applications, PMLR, Oct. 2017, pp. 152-163. Accessed: Jul. 29, 2023. [Online]. Available: https://proceedings.mlr.press/v74/fayet17a.html
[92] F. Ye and J. Yang, "A Deep Neural Network Model for Speaker Identification," Appl. Sci., vol. 11, no. 8, Art. no. 8, Jan. 2021, doi: 10.3390/app11083603.
[93] Z. Li, Z. Han, M. Huang, and L. Kong, "An Ensemble Machine Learning Classifier for Profiling Irony and Stereotype Spreaders on Twitter," in CEUR Workshop Proceedings, Bologna, Italy, 2022.
[94] O. Eriksson, B. Henderson-Sellers, and P. J. Agerfalk, "Ontological and linguistic metamodelling revisited: A language use approach," Inf. Softw. Technol., vol. 55, no. 12, pp. 2099-2124, Dec. 2013, doi: 10.1016/j.infsof.2013.07.008.
[95] M. Sukhareva and C. Chiarcos, "An Ontology-based Approach To Automatic Part-of-Speech Tagging Using Heterogeneously Annotated Corpora," in Proceedings of the Second Workshop on Natural Language Processing and Linked Open Data, Hissar, Bulgaria: INCOMA Ltd. Shoumen, BULGARIA, Sep. 2015, pp. 23-32. Accessed: Jul. 29, 2023. [Online]. Available: https://aclanthology.org/W15-5505
[96] S. Nirenburg and V. Raskin, Ontological Semantics. Cambridge, Mass: The MIT Press, 2004.
[97] C. Panayiotou, "An Ontological Analysis and Natural Language Processing of Figures of Speech," Int. J. Artif. Intell. Appl., vol. 11, no. 1, pp. 17-30, Jan. 2020, doi: 10.5121/ijaia.2020.11102.
[98] G. Deepak, D. Surya, I. Trivedi, A. Kumar, A. Lingampalli, and S. vijayan, "An artificially intelligent approach for automatic speech processing based on triune ontology and adaptive tribonacci deep neural networks," Comput. Electr. Eng., vol. 98, p. 107736, Mar. 2022, doi: 10.1016/j.compeleceng.2022.107736.
[99] M. Chen, "Using Ontology-based Approaches to Representing Speech Transcripts for Automated Speech Scoring," in Proceedings of the NAACL HLT 2012 Student Research Workshop, Montréal, Canada: Association for Computational Linguistics, Jun. 2012, pp. 41--47. Accessed: Jul. 29, 2023. [Online]. Available: https://aclanthology.org/N12-2008
[100] N. Madnani and A. Cahill, "Automated Scoring: Beyond Natural Language Processing," in Proceedings of the 27th International Conference on Computational Linguistics, Santa Fe, New Mexico, USA: Association for Computational Linguistics, Aug. 2018, pp. 1099-1109. Accessed: Jul. 29, 2023. [Online]. Available: https://aclanthology.org/C18-1094
[101] M. R. Iseli, A. D. Koenig, J. J. Lee, and R. Wainess, "Automated assessment of complex task performance in games and simulations," in Proceedings of the Interservice/Industry Training, Simulation and Education Conference, Orlando, FL, Orlando, FL, 2010.
[102] D. M. Williamson, R. G. Almond, R. J. Mislevy, and R. Levy, "An Application of Bayesian Networks in Automated Scoring of Computerized Simulation Tasks," in Automated scoring of complex tasks in computer-based testing, Mahwah, NJ, US: Lawrence Erlbaum Associates Publishers, 2006, pp. 201-257.
[103] M. A. Petilli, R. Daini, F. L. Saibene, and M. Rabuffetti, "Automated scoring for a Tablet-based Rey Figure copy task differentiates constructional, organisational, and motor abilities," Sci. Rep., vol. 11, no. 1, p. 14895, Jul. 2021, doi: 10.1038/s41598-021-94247-9.
[104] P. Deane, T. Quinlan, and I. Kostin, "AUTOMATED SCORING WITHIN A DEVELOPMENTAL, COGNITIVE MODEL OF WRITING PROFICIENCY," ETS Res. Rep. Ser., vol. 2011, no. 1, pp. i-93, Jun. 2011, doi: 10.1002/j.2333-8504.2011.tb02252.x.
[105] F. Roux, B. C. Armstrong, and M. Carreiras, "Chronset: An automated tool for detecting speech onset," Behav. Res. Methods, vol. 49, no. 5, pp. 1864-1881, Oct. 2017, doi: 10.3758/s13428-016-0830-1.
[106] J. McKechnie, B. Ahmed, R. Gutierrez-Osuna, P. Monroe, P. McCabe, and K. J. Ballard, "Automated speech analysis tools for children's speech production: A systematic literature review," Int. J. Speech Lang. Pathol., vol. 20, no. 6, pp. 583-598, Oct. 2018, doi: 10.1080/17549507.2018.1477991.
[107] P. A. Jansen and S. Watter, "SayWhen: An automated method for high-accuracy speech onset detection," Behav. Res. Methods, vol. 40, no. 3, pp. 744-751, Aug. 2008, doi: 10.3758/BRM.40.3.744.
[108] J. Zeremdini, M. A. Ben Messaoud, and A. Bouzid, "A comparison of several computational auditory scene analysis (CASA) techniques for monaural speech segregation," Brain Inform., vol. 2, no. 3, pp. 155-166, Sep. 2015, doi: 10.1007/s40708-015-0016-0.
[109] A. S. Bregman, Auditory Scene Analysis: The Perceptual Organization of Sound. The MIT Press, 1994.
[110] D. Stowell, D. Giannoulis, E. Benetos, M. Lagrange, and M. D. Plumbley, "Detection and Classification of Acoustic Scenes and Events," IEEE Trans. Multimed., vol. 17, no. 10, pp. 1733-1746, Oct. 2015, doi: 10.1109/TMM.2015.2428998.
[111] S. Teki, M. Chait, S. Kumar, S. Shamma, and T. D. Griffiths, "Segregation of complex acoustic scenes based on temporal coherence," eLife, vol. 2, p. e00699, Jul. 2013, doi: 10.7554/eLife.00699.
[112] I. Winkler, S. L. Denham, and I. Nelken, "Modeling the auditory scene: predictive regularity representations and perceptual objects," Trends Cogn. Sci., vol. 13, no. 12, pp. 532-540, Dec. 2009, doi: 10.1016/j.tics.2009.09.003.
[113] I. Nelken, "Processing of complex stimuli and natural scenes in the auditory cortex," Curr. Opin. Neurobiol., vol. 14, no. 4, pp. 474-480, Aug. 2004, doi: 10.1016/j.conb.2004.06.005
[114] D. Wang and J. Chen, "Supervised Speech Separation Based on Deep Learning: An Overview," IEEEACM Trans. Audio Speech Lang. Process., vol. 26, no. 10, pp. 1702-1726, Oct. 2018, doi: 10.1109/TASLP.2018.2842159.
[115] "Jose J. Lopez | Home page." http://personales.upv.es/jjlopez/SSS.html (accessed Jul. 28, 2023).
[116] K. Cao and M. Wang, "Transient noise suppression algorithm in speech system," AIP Conf. Proc., vol. 1864, no. 1, p. 020006, Aug. 2017, doi: 10.1063/1.4992823.
[117] P. Korhonen, F. Kuk, C. Lau, D. Keenan, J. Schumacher, and J. Nielsen, "Effects of a transient noise reduction algorithm on speech understanding, subjective preference, and preferred gain," J. Am. Acad. Audiol., vol. 24, no. 9, pp. 845-858, Oct. 2013, doi: 10.3766/jaaa.24.9.8.
[118] H. R. Staff, "Comparison of Transient Noise Reduction Systems," The Hearing Review, Jan. 03, 2008. https://hearingreview.com/practice-building/practice-management/comparison-of-transient-noise-reduction-systems (accessed Jul. 22, 2023).
[119] M.-S. Choi and H.-G. Kang, "Transient noise reduction in speech signal with a modified long-term predictor," EURASIP J. Adv. Signal Process., vol. 2011, no. 1, p. 141, Dec. 2011, doi: 10.1186/1687-6180-2011-141.
[120] R. Ullah, M. S. Islam, Z. Ye, and M. Asif, "Semi-supervised transient noise suppression using OMLSA and SNMF algorithms," Appl. Acoust., vol. 170, p. 107533, Dec. 2020, doi: 10.1016/j.apacoust.2020.107533.
[121] S. Graf, T. Herbig, M. Buck, and G. Schmidt, "Features for voice activity detection: a comparative analysis," EURASIP J. Adv. Signal Process., vol. 2015, no. 1, p. 91, Dec. 2015, doi: 10.1186/s13634-015-0277-z.
[122] M. Sharma, S. Joshi, T. Chatterjee, and R. Hamid, "A comprehensive empirical review of modern voice activity detection approaches for movies and TV shows," Neurocomputing, vol. 494, pp. 116-131, Jul. 2022, doi: 10.1016/j.neucom.2022.04.084.
[123] J. Lian, A. Baevski, W.-N. Hsu, and M. Auli, "AV-data2vec: Self-supervised Learning of Audio-Visual Speech Representations with Contextualized Target Representations." arXiv, Feb. 09, 2023. doi: 10.48550/arXiv.2302.06419.
[124] A. Baevski, A. Babu, W.-N. Hsu, and M. Auli, "Efficient Self-supervised Learning with Contextualized Target Representations for Vision, Speech and Language," in Proceedings of the 40 th International Conference on Machine Learning, Honolulu, Hawaii, USA, 2023.
[125] A. Haliassos, P. Ma, R. Mira, S. Petridis, and M. Pantic, "JOINTLY LEARNING VISUAL AND AUDITORY SPEECH REPRESENTATIONS FROM RAW DATA," presented at the ICLR, 2023.
[126] Y. Hu, C. Chen, R. Li, H. Zou, and E. S. Chng, "MIR-GAN: Refining Frame-Level Modality-Invariant Representations with Adversarial Network for Audio-Visual Speech Recognition," in Proceedings of the 61st Annual Meeting of the Association for Computational Linguistics (Volume 1: Long Papers), Toronto, Canada: Association for Computational Linguistics, Jul. 2023, pp. 11610-11625. Accessed: Jul. 28, 2023. [Online]. Available: https://aclanthology.org/2023.acl-long.649
[127] M. Pastor, D. Ribas, A. Ortega, A. Miguel, and E. Lleida, "Cross-Corpus Speech Emotion Recognition with HuBERT Self-Supervised Representation," in IberSPEECH 2022, ISCA, Nov. 2022, pp. 76-80. doi: 10.21437/IberSPEECH.2022-16.
[128] P. Ma, A. Haliassos, A. Fernandez-Lopez, H. Chen, S. Petridis, and M. Pantic, "Auto-AVSR: Audio-Visual Speech Recognition with Automatic Labels," ICASSP 2023 - 2023 IEEE Int. Conf. Acoust. Speech Signal Process. ICASSP, pp. 1-5, Jun. 2023, doi: 10.1109/ICASSP49357.2023.10096889.
[129] B. Shi, W.-N. Hsu, and A. Mohamed, "Robust Self-Supervised Audio-Visual Speech Recognition," Interspeech 2022, pp. 2118-2122, Sep. 2022, doi: 10.21437/Interspeech.2022-99,
[130] B. Shi, W.-N. Hsu, K. Lakhotia, and A. Mohamed, "Learning Audio-Visual Speech Representation by Masked Multimodal Cluster Prediction," ArXiv, Jan. 2022, Accessed: Jul. 28, 2023. [Online]. Available: https://www.semanticscholar.org/paper/Learning-Audio-Visual-Speech-Representation-by-Shi-Hsu/dc9a76b7cb690e6a95f0f07bb3d4fabb7181b68d
[131] W.-N. Hsu, B. Bolte, Y.-H. H. Tsai, K. Lakhotia, R. Salakhutdinov, and A. Mohamed, "HuBERT: Self-Supervised Speech Representation Learning by Masked Prediction of Hidden Units," IEEEACM Trans. Audio Speech Lang. Process., vol. 29, pp. 3451-3460, Oct. 2021, doi: 10.1109ITASLP.2021.3122291.
[132] W.-N. Hsu and B. Shi, "u-HuBERT: Unified Mixed-Modal Speech Pretraining And Zero-Shot Transfer to Unlabeled Modality".
[133] "232431_GIANNETTI_JACOPO.pdf." Accessed: Jul. 28, 2023. [Online]. Available: http:/itesi.luiss.it/30515/1/232431_GIANNETT_JACOPQ.pdf
[134] C. D. Manning, K. Clark, J. Hewitt, U. Khandelwal, and O. Levy, "Emergent linguistic structure in artificial neural networks trained by self-supervision," Proc. Natl. Acad. Sci., vol. 117, no. 48, pp. 30046-30054, Dec. 2020, doi: 10.1073/pnas.1907367117.
[135] V. Robles-Bykbaev, J. J. Pazos-Arias, M. López-Nores, J. Garcia-Duque, and J. Ochoa-Zambrano, "Modelling Domain Knowledge of Speech and Language Therapy with an OWL Ontology and OpenEHR Archetypes:," in Proceedings of the International Conference on Health Informatics, Lisbon, Portugal: SCITEPRESS - Science and and Technology Publications, 2015, pp. 585-591. doi: 10.5220/0005279405850591.
[136] U. Jekosch, Voice and Speech Quality Perception: Assessment and Evaluation, 2005th edition. Berlin ; New York: Springer, 2005.
[137] P. Martin, Speech Acoustic Analysis, 1st edition. Hoboken: Wiley-ISTE, 2021.
[138] J. Kreiman, Y. Lee, M. Garellek, R. Samlan, and B. R. Gerratt, "Validating a psychoacoustic model of voice quality," J. Acoust. Soc. Am., vol. 149, no. 1, pp. 457-465, Jan. 2021, doi: 10.1121/10.0003331.
[139] G. E. Henter, "Probabilistic Sequence Models with Speech and Language Applications".
[140] Y. Bengio, R. Ducharme, P. Vincent, and C. Jauvin, "A Neural Probabilistic Language Model".
[141] L. Li, R. Rehr, P. Bruns, T. Gerkmann, and B. Röder, "A Survey on Probabilistic Models in Human Perception and Machines," Front. Robot. AI, vol. 7, 2020, Accessed: Jul. 29, 2023. [Online]. Available: https://www.frontiersin.org/articles/10.3389/frobt, 2020.00085
[142] V. Y. Davier Matthias von, Ed., Advancing Natural Language Processing in Educational Assessment. New York: Routledge, 2023. doi: 10.4324/9781003278658.
[143] N. Harris, "Interpretability Methods in Machine Learning: A Brief Survey," Two Sigma. https://www.twosigma.com/articles/interpretability-methods-in-machine-learning-a-brief-survey/ (accessed Jul. 29, 2023).

## Claims

1. A computer-implemented method executed by a processor for the acquisition, assessment and scoring of a subject's speech under a set of tasks related to cognition and speech production in speech-related cognitive and motor processes, **characterized in that** the method comprises the following steps:
a data and response step (110), for collecting the subject's multimodal raw data related to task, wherein said raw data comprises stimulation/testing data regarding speech-related responses and Electronic Health Record (EHR) data;
a speech extraction step (120), for extracting the subject's speech/voice signals from the collected raw data by detecting, identifying, discriminating and isolating the subject's speech from unrelated signals, and generating a clean signal;
an utterance processing step (130), for analyzing the extracted speech signal to generate a detailed utterance structure,
wherein said detailed utterance structure is evaluated and quantified, by a first assessment and indices function (141), against a set of predetermined task/paradigm targets for yielding a characterization and scoring for the subject's performance, and simultaneously modeled, by a modeling and profiling module (142), for generating profiles of utterance specific, subject specific, population specific performance, and speech characteristics, based on a set of parameters,
wherein the generated profiles is used both as an autonomous output and as feedback to the utterance processing step (130) for enhancing accuracy and speed of the process, and the result of analysis and the generated profiles are fed back to a processing unit (150) which evaluates, in real-time, the predetermined targets and the set of parameters for maximizing the informational content of the generated profiles at the subject level and/or at the population level,
a database storage step (160) for storing the data, inputs and outputs, in a local and/or remote database storage unit (160); and
wherein the method further includes a training and supervision step (100), to fully train and supervise all the steps of the method.

2. Computer-implemented method of collection and automated analysis, evaluation and scoring of speech of a subject, in particular according to claim 1, which is executed on a computer system comprising processor, memory, input/output units and database, within a set of tasks - tests related to cognitive function and speech production, rehabilitation of speech-language disorders and intervention devices such as brain stimulation devices (TMS/DCS), intended for rehabilitation, training and evaluation of speech, **characterized in that** it includes the following steps executed through said computer system:
- a data and response step (110), where through sensors and computational interfaces structured multimodal primary data of the subject are collected which relate to task-process-work and/or stimulation/examination regarding responses related to speech (110) and especially together with structured Electronic Health Record (EHR) data and structured data of test-process-work/paradigm/stimulation, where said data are used in the subsequent steps (120 ...),
- a speech extraction step (120), implemented by a signal processing unit, where the speech/voice signals of the subject are detected (120), then recognized, further distinguished and isolated from irrelevant signals, mainly noise, or other speech, creating a digital clean signal which is further fed into a next step (130);
- a further speech processing stage (130), implemented by a computational processing unit and speech-recognition software, where the extracted speech signal is processed and analyzed (130), producing and extracting a detailed utterance structure, including timings, phonetic, lexical, semantic and pragmatic features;
- a further functional step (140), where two functions are executed in parallel (140) consisting of a first evaluation and indices function (141), where said detailed utterance structure (130) and the events are evaluated and quantified against a set of predefined targets of task-process/paradigm, providing characterization and scoring for the performance of a specific subject, and a second modeling and profiling function (142), where the same detailed speech structure (130) and events are simultaneously fed into a modeling-classification-profiling block (142) constituting a machine learning (ML) sub-module implemented in software on the processor of the system, which applies classifiers or regression/prediction models based on techniques such as Gradient Boosting Machines (XGBoost), Random Forests or Support Vector Machines (SVM), so as to identify non-linear patterns and create performance profiles of specific utterance, specific subject and specific population, as well as speech characteristic profiles, in combination with a set of parameters, where this profile is used as an autonomous output and as feedback to the above speech processing step (130) to improve the accuracy and speed of the process, as well as software function, structure and implementation whereby the ML model is trained and readjusted in real time, receiving data from the database (160) of the system, while its parameters and hyper-parameters are automatically computed and optimized;
- a process coordination step (150), where the results of analysis and profile (142) are fed back for processing, implemented by a processing and control unit in the form of computer software (150) which evaluates in real time the produced profiles and adjusts the parameters and target conditions for the following steps (110 ...), including (i) the conditions of stimulus presentation -such as. time, type, of images/sounds/other stimuli-, and (ii) the settings of interventional brain stimulation devices -such as TMS, DCS,-, thereby providing a real adaptation of the physical parameters of those devices through the outputs of the computing system, esp. for maximizing the informational content of the profiles at individual subject or population level;
- an additional database storage step (160) where all data, inputs and outputs of the previous stages are exchanged, in all steps (110 ...), with a local and/or remote database storage unit (160) of the system and are used for feedback re-training of the ML model, ensuring continuous optimization of prediction accuracy and adaptive settings; and
- a training and supervision step (100) through a computing training unit in which the system is fully trainable and supervisable in all the preceding steps (110 ...), through a remote or local computer interface or cloud infrastructure.

3. The computer-implemented method according to claim 1 or 2, **characterized in that** said tasks comprise frameworks of complex tasks related to cognition and speech production, rehabilitation for Speech-Language Disorders, and interventional setups, and
wherein the subjects' multimodal raw data comprises speech/voice recordings, video recordings of facial/motional/positional/gestural state, vibratory state, neuro-muscular activity, and
wherein said detailed utterance structure includes transcribed content, timings, phonetics, and identification of singular events consisting of hesitations, elongations; and
wherein the set of parameters comprises health parameters.

4. The computer-implemented method according to one of the claims 1 to 3, **characterized in that** said frameworks comprises pictures, sounds, and parameters regarding external interventions to the subject's state selected from Transcranial Magnetic Stimulation (TMS), DCS, or a stimulation/disturbance comprising of intensities, patterns, timings, spatial/volumetric distributions, and wherein said processing unit (150) determines the content characteristics, presentation levels, timings of said subject's sensory templates.

5. The computer-implemented method according to one of the claims 1 to 4, **characterized in that** the Speech-related Responses are analyzed and profiled in a Picture Naming Task under Transcranial Magnetic Stimulation (TMS), wherein said task is defined as the subject's ability to name pictures under the (TMS) stimulation and is assessed for speech production structures, wherein said assessment comprises :
a first phase (ϕ1) of acquiring the subject's baseline performance characteristics based on the responses recorded and analyzed for the pictures presented to the subject without including said TMS stimulation, to obtain reference performance values and information on appropriate parameterization of the process, and
a second phase (ϕ₂) for mapping brain paradigm using the (TMS) stimulation, based on the results of the first phase (ϕ₁).

6. The computer-implemented method according to claim 5, **characterized in that** the first phase (ϕ₁) further comprises:
Step (a) of determining the pictures associated to abnormal responses by the subject and removing said determined pictures are removed from the presentation material of the subsequent actual mapping of the second phase (ϕ₂), and
Step (b) of adjusting the interpicture interval (IPI) and the picture presentation time (PPT) to fit the subject's performance ability,
wherein the step (a) includes an automated process of cleaning recorded responses from undesired intruding signals, together with speech analysis and recognition for yielding the identification of abnormal responses and the step (b) includes a customized algorithmic for adjustment of said interpicture interval (IPI) and the picture presentation time (PPT), wherein said response are registered at each trial, and the the values for each said responses forms the distribution of the characteristic at normal response for using as a template for categorizing responses as normal or abnormal, and
wherein the normal utterance/speech characteristic distributions are subject-specific and adapted to the designed interpicture interval (IPI) and the picture presentation time (PPT).

7. The computer-implemented method according to claim 5 or 6, **characterized in that** the pictures not removed from the first phase (ϕ1) is provided to the subject in said second phase (ϕ₂), , and the (TMS) stimulation is performed over a section of the cortical region in the perisylvian area, with a dense spatial sampling perilesionally, esp. in synchrony with the appearance of pictures,
particularly wherein a picture-to-TMS trigger interval (PTI) = 0 ms, and each brain hemisphere is stimulated in a range of 40 to 80 stimulation sites at a sequence,
more particularly wherein each stimulation site is stimulated in nonconsecutive times, esp. three, resulting in a total of 120 to 240 stimulations per brain hemisphere, and includes both the lesion-harboring brain hemisphere as well as the contralateral one, and identify lesion-induced plasticity phenomena based on the response to each said stimulus classified as either normal or abnormal, wherein the abnormal responses being further classified as no response (speech arrest or aphasic anomia), performance error (including dysarthria or speech apraxia), hesitation, circumlocution, paraphasias (semantic or phonological), and neologisms.

8. The computer-implemented method according to claim 7, **characterized in that** the stimulation paradigm in said second phase (ϕ₂) is adaptive, and full parametrization of the stimulation paradigm comprises stimulus intensity and frequency, number of stimuli, the IPI, PPT and PTI, esp. based on the results of the first phase (ϕ1) and on the speed and accuracy of the automated extraction of results, to identify abnormal responses, and customization of parametrization of the full set of stimulation parameters.

9. The computer-implemented method according to one of the claims 5 to 8, **characterized in that** said first phase (ϕ₁) and said second phase (ϕ₂), are further trained to assign threshold values defining distribution tails of each normal uttering/speech characteristic wherein said threshold values determine the deviation from normal of each speech characteristic and classify in terms of each characteristic the response as normal or abnormal,
particularly wherein the subject's responses, from said second phase (ϕ₂) are analyzed and each response is again classified as normal or abnormal, wherein the abnormal responses further classified as "no response", "performance error", hesitation, circumlocution, paraphasias and neologisms, and wherein every brain stimulation site where an error is identified on at least two nonconsecutive times is regarded as nr TMS-positive and indicates a functional, language-related brain tissue.

10. The computer-implemented method according to one of the claims 5 to 9, **characterized in that** the identification of abnormal responses is performed in real-time, for identification of abnormal responses and a detailed subject's utterance profile, to obtain the construction of a subject's language network brain map, particularly wherein fusion schemes are realized based on outcomes of the decision whether each uttering/speech characteristic is normal or abnormal- at each of three repetitions, to determine the overall response as normal or abnormal, and assign a score of abnormality based on the decision on each of the specific abnormality types, selected from "no response", "performance error", hesitation, circumlocution, paraphasias and neologisms.

11. Computer system configured for the execution of a method as defined in one of the preceding claims 1 to 10, mainly through an integrated, automated software/hardware which operates in a cooperative manner with regard to the interconnections between blocks/modules/units and/or in cooperation with human operators, **characterized in that** it comprises a data-means unit (110) for the acquisition of multimodal responses of the subject as well as the collection of data of the subject, which is interactively interconnected with a speech-extraction-means unit (120) for the distinction and isolation of the speech and voice of the subject from irrelevant activity or signals, which in turn is further connected with a speech-processing-means unit (130) for the analysis and recognition of speech, which in turn is further connected with a functional-means unit (140) consisting of a means unit of evaluation and indices (141) for the evaluation of the utterance according to the tests/procedures/processes of the paradigm and the estimation of indices as a first functional unit on one hand, and on the other hand of a functional unit of modeling and profile creation of the utterance and of the subject as a second unit (142) which is connected with the aforementioned first functional unit (141), where the above-mentioned second functional unit (141) is recursively connected with the aforesaid speech-processing-means unit (130), where it further comprises a process-coordination-means unit (150) which is interactively interconnected with the said functional-means unit (140) and the aforesaid data and response-means unit (110), including a recursive connection (g, h) with it (110, 140), where additionally a database-storage-means unit (160) is provided, which is connected with the said data-means unit (110), the said speech-extraction-means unit (120), the said speech-processing-means unit (130) and the said functional-means unit (140) respectively, and where a training and supervision means unit (100) is also provided, which is interactively interconnected with the said data and response-means unit (110), the said speech-extraction-means unit (120), the said speech/utterance processing unit (130) and the said functional-means unit (140) respectively, where the cooperation of the units is implemented through software and hardware processors, and the system produces technical effect by controlling real stimulus presentation and stimulation devices.

12. System according to claim 11, which is executed by a processor or GPU/TPU on a computing platform or cloud environment, for the Collection resp. Acquisition and Automated Analysis, Assessment resp. evaluation and Scoring of Subjects' speech, under a set of tasks comprising Frameworks of complex tasks related to cognition and speech production, Rehabilitation for Speech-Language Disorders- and interventional setups, in the form of a computer and network notably intended for rehabilitation, training and assessment of speech, wherein it comprises a set of operating means for a collective and integrated methodological and functional/operational flow of data means and a system's synthesis means for an integrated and automated software/hardware system which performs interactively in terms of interconnections between modules (I1,..., I3; A, B,..., L) and/or in cooperation with operators, the said operations means comprising
- Collection means (I1) of subject's responses, along with a set of additional data means (I2),
- Removal means (C) of undesired signals (UW) from the subject's speech,
- Means (D) for the performance of phonetical, phonological, lexical, semantic, and pragmatic NLP along or after the speech's content recognition,
- Means for the Performance of intelligent and adaptive coordination of execution flow and selection of the task presentation and interventional parameters,
- Building means (E) for building a system structure that is trainable and supervisable (100), which recurrently accumulates, analyses and makes decisions upon reception of an input resp. output, esp. thereby generating a so-called smart system.

13. A computer-implemented system for carrying out a method as defined in one of the claims 1 to 10, wherein the system comprises:
a data means unit (110) for the acquisition of subject's responses and the subject's data, and is interactively interconnected with a speech extraction means unit (120) for collecting a subject's speech and isolate the subject's voice from unrelated activity or signals,
an utterance processing means unit (130) connected to the speech extraction means unit (120) for utterance analysis and recognition,
a functional means unit (140) connected to the utterance processing means unit (130) consisting of:
an assessment and indices means unit (141) for utterance assessment according to paradigm tasks and estimation of indices as first functional unit, and
a modelling and profiling means unit (142) connected to said first functional unit (141) for utterance and subject's modelling and profiling as second functional unit, wherein said second functional unit (141) is connected retroactively with the utterance processing means unit (130),
a process coordination means unit (150) interconnected with said functional means unit (140) and the data and response means unit (110),
a database storage means unit (160) connected to said data means unit (110), said speech extraction means unit (120), said utterance processing means unit (130) and said functional means unit (140), and a training and supervision means unit (100) interconnected with said data and response means unit (110), said speech extraction means unit (120), said utterance processing means unit (130) and said functional means unit (140).

14. System according to one of the claims 11 to 13, esp. the latter, **characterized in that** it further comprises a set of operating means for a collective and integrated flow of data means and a system's synthesis means for an integrated and automated software/hardware system, wherein the said set of operating means comprising:
a collection means (I1) of subject's responses, along with a set of additional data means (12),
a removal means (C) of undesired signals (UW) from the subject's speech,
a means (D) for the performance of phonetical, phonological, lexical, semantic, and pragmatic NLP along or after the speech's content recognition,
a means for the Performance of intelligent and adaptive coordination of execution flow and selection of the task presentation and interventional parameters,
a building means (E) for building a system structure that is trainable and supervisable (100), which recurrently accumulates, analyses and makes decisions upon reception of an input resp. output, thereby generating a so-called smart system.

15. System according to claim 14, **characterized in that** it further comprises a plurality of input ports (I1, I2, I3) comprising:
a primary main input stream (I1) constituted by raw signal data collected from the subject, as well as task specific data/metadata, and undesirable signals (UW),
an accessory input stream (I2) constituted by additional data apart from said primary input stream (11), an input module (I3) of task descriptors and targets predefining quantitative and qualitative indices of interest as well as the targets of a specific experimental paradigm,
wherein the system further includes an analysis means (A) for performing an analysis of a subject's scene, esp. for identification of events that are captured by a set of sensors and obtained from said primary input stream (I1), wherein the analysis of events regarding the subject's responses and/or an additional external event taking place in the subject's scene is performed in said analysis means (A) to identify and classify the events related to the information contained in said primary input stream (I1), and said classified data are fed to a filtration means (B), wherein a discrimination is performed between target-of-interest (TOI) responses/reactions from the subject and other eventful signals that are not of interest constituting Unwanted signals (UWs), for isolating the target-of-interest (TOI) for further analysis,
wherein the Unwanted signals (UWs) are eliminated, to obtain a clarified target-of-interest (TOI) that contains a subject's isolated and purified utterance speech for the subsequent analyses,
wherein a detection means (D1) is used for detecting, identifying, classifying and segregating the remaining cleaned parts of the utterance in terms of phonemic/phonological units, wherein a logical classification of content as events is performed, providing a deliverable at the output that is readily available, particularly as a table with timings and nature of the utterance,
wherein the speech units -of which silence is excluded- identified in said detection means (D1) are transcribed into phonetic symbols and strings in transcription means (D2), wherein morphological analysis, concurrent lexical, syntactic, semantic, and pragmatic analysis and annotation of the whole subject's utterance is provided by a main processing unit (D3), wherein It recognizes discrete behavioral sequences of speech and language units and recognizes the target word/phrase through a syntactic/pragmatic analysis,
wherein the main processing unit (D3) is provided for producing probabilistic hierarchies of results that contain the identifications and marks of the said parts of the utterance that correspond to the subject's intentioned response to the task, wherein sequenced, layered, object oriented ontological models of the said utterance are built by said building means (E), thereby generating deliverables readily readable in a lecture format, particularly by tables, for use in a profile processor means (G), wherein a phonetic analysis of each part of the utterance is performed in said phonetic analyzer means (F), by identifying and quantifying any phonetic parameters of interest, wherein data, inputs and outputs are stored in a database means (K) -local or remote or mixed- and exchanged with above-mentioned means especially (D1, F, G,..).

16. System according to claim 15, **characterized in that** it further comprises additional processor means (J) to take input from said processor unit (D3), said phonetic analyzer means (F) and task targets to quantify the various parameters of interest (POI) as well as the degree of conformance in terms of accuracy or repeatability, namely types and numbers of divergences/errors from the specified task targets,
wherein a process coordinator means (L) in connection with said additional input module (I3) and said additional processor means (J) adaptively coordinates the task's/paradigm's presentation and/or intervention parameters, according to specific statistical or informational rules/targets, particularly wherein said process coordinator means (L) suggests the values of the stimulation parameters and the parameters or content of the templates presented to the subject, such as sounds, pictures, resp. or even directly controls the software/hardware means performing the stimulation and template presentation.

17. System according to claim 16, **characterized in that** said additional processor means (J) outputs a table with timings and nature of the utterance, particularly wherein additionally, prior statistical knowledge of the subject's speech profile is also incorporated as feedback from phonetic analyzer means (F),
more particularly wherein, the processor unit (D3) outputs a complete table of fully time marked, transcribed, lexically, syntactically, semantically and pragmatically annotated written content of the subject's utterance, preferably wherein said processor unit (D3) operates on the audio of the speech signal, as well as in a combined stream of multimodal signals esp. originating from said input module (11), such as audio-visual esp, audio together with video recording of gestural, facial, motional states, vibrational and neuro-muscular.

18. System according to one of the claims 15 to 17, **characterized in that** the analysis in modules (D) is upgraded, with the registered and exchanged information with a database modules (K), wherein the database modules stores information in the form of records and fields or as object-oriented structures based on information collected from said input modules (I2, I3), and generated by the modules (K, E, F) selected from the subject's demographics, session place/time/duration details, paradigm/task parameters/components/templates, interventional parameters/ components/templates, wherein the profiles are created by combining and aggregating any of the aforementioned information types.

19. System according to claim 18, **characterized in that** the profiles are continuously updated with the accumulation of new data and analysis results from said modules (D, E, F), such as in each presentation during a repetitive naming task, and incorporating a decision-making step such as maximum likelihood selection, the module's output is fed back to said modules (D) for further improving their statistical performance, in terms of prior statistical knowledge of the subject's speech profiles.

20. Method for the acquisition analysis, assessment and Scoring of a subjects' speech as defined in one of the claims 1 to 10, performed on a system as defined in one of the claims 11 to 19, wherein the method comprises a variety of paradigms/tasks for clinical/non-clinical research, rehabilitation, training and assessment of speech.

21. Use of the method according to one of the claims 1 to 10 or 20, for an automated profiling of speech responses in mapping and rehabilitation of speech, **characterized in that** it is based on the following steps :
- performing automatically and fast with minimal human operator effort & intervention;
- extracting, quantifying, evaluating and assessing the subject's speech and/or its characteristics such as accuracy, quantification of diversions or adherence or conformance to the demanded task, namely errors, semantics, lexical, pragmatics, phonetics, timings, sequential characteristics, of the response) in relation to the offered visual, auditory, olfactory, tactile, vibratory, motional template and demanded task;
- performing classification, clustering and modeling of the content and signal characteristics of the sequences of events in the subject responses, producing appropriate utterance's and speaker's profiles;
- augmenting the analysis of the latter step by an appropriate feedback from previous utterance's/speaker's profiles, which are formed by accumulation of previous analyses or beforehand knowledge;
- maximizing and optimizing the acquired subject or population profile information through an adaptive coordination of the process; by means of
a set of proposed operations consisting of
- the Collection of subject responses, along with a variety of additional data;
- the Removal of any unwanted signals that may be mixed with the subject speech;
- the Performance of phonetical, phonological, lexical, semantic, and pragmatic NLP along/or after the speech's content recognition;
- the Performance of intelligent and adaptive coordination of execution flow and selection of the task/paradigm's presentation and interventional parameters;
- the Building of a system that is structured to be trainable and supervisable, esp. that recurrently accumulates, analyses and makes decisions upon its inputs and outputs, and thus constitutes an intelligent system.
